(19) **Europäisches Patentamt**
**European Patent Office**
**Office européen des brevets**

(11) **EP 3 064 221 A1**

(12) **EUROPEAN PATENT APPLICATION**

(43) Date of publication:
07.09.2016 Bulletin 2016/36

(51) Int Cl.:
*A61K 39/395* (2006.01)    *C07K 16/24* (2006.01)
*A61K 31/7068* (2006.01)    *A61K 31/704* (2006.01)
*A61K 33/24* (2006.01)    *A61K 31/137* (2006.01)
*A61P 35/00* (2006.01)    *A61K 31/136* (2006.01)
*A61K 39/00* (2006.01)

(21) Application number: 16165526.1

(22) Date of filing: 16.04.2013

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME

(30) Priority: 16.04.2012 US 201261624964 P
06.07.2012 US 201261668932 P
12.03.2013 US 201361778094 P

(83) **Declaration under Rule 32(1) EPC (expert solution)**

(62) Document number(s) of the earlier application(s) in accordance with Art. 76 EPC:
**13728681.1 / 2 838 561**

(71) Applicants:
• **Baxalta GmbH**
**8152 Glattpark, Opfikon (CH)**

• **Baxalta Incorporated**
**Bannockburn, IL 60015 (US)**

(72) Inventors:
• **Kerschbaumer, Randolf**
**3400 Klosterneuburg (AT)**
• **Scheiflinger, Friedrich**
**1090 Wien (AT)**
• **Ehrlich, Hartmut**
**1170 Wien (AT)**

(74) Representative: **Hoffmann Eitle**
**Patent- und Rechtsanwälte PartmbB**
**Arabellastraße 30**
**81925 München (DE)**

Remarks:
This application was filed on 15-04-2016 as a divisional application to the application mentioned under INID code 62.

(54) **COMBINATION THERAPY OF ANTI-MIF ANTIBODIES AND CHEMOTHERAPEUTICS**

(57)    The present invention pertains to anti-MIF antibodies, in particular their use in combination with cancer therapeutics, i.e. chemotherapeutics, in the treatment of cancer.

Figure 1

% Increase of caspase 3 activity — A
(Control IgG, RAM0, Control IgG + Dox, Dox, RAM0 + Dox)

% Increase of caspase 3 activity — B
(Control IgG, RAM9, Control IgG + Dox, Dox, RAM9 + Dox)

EP 3 064 221 A1

SEQUENCE LISTING

<110> Baxter Healthcare S.A.
Baxter International Inc.

<120> COMBINATION THERAPY OF ANTI-MIF ANTIBODIES AND CHEMOTHERAPEUTICS

<130> 163 259

<160> 14

<170> PatentIn version 3.5

<210> 1
<211> 214
<212> PRT
<213> Artificial Sequence

<220>
<223> Light chain of RAB9

<400> 1

Asp Ile Gln Met Thr Gln Ser Pro Ser Ser Leu Ser Ala Ser Val Gly
1               5                   10                  15


Asp Arg Val Thr Ile Thr Cys Arg Ser Ser Gln Arg Ile Met Thr Tyr
            20                  25                  30


Leu Asn Trp Tyr Gln Gln Lys Pro Gly Lys Ala Pro Lys Leu Leu Ile
            35                  40                  45


Phe Val Ala Ser His Ser Gln Ser Gly Val Pro Ser Arg Phe Arg Gly
        50                  55                  60


Ser Gly Ser Glu Thr Asp Phe Thr Leu Thr Ile Ser Gly Leu Gln Pro
65                  70                  75                  80


Glu Asp Ser Ala Thr Tyr Tyr Cys Gln Gln Ser Phe Trp Thr Pro Leu
                85                  90                  95


Thr Phe Gly Gly Gly Thr Lys Val Glu Ile Lys Arg Thr Val Ala Ala
            100                 105                 110


Pro Ser Val Phe Ile Phe Pro Pro Ser Asp Glu Gln Leu Lys Ser Gly
            115                 120                 125


Thr Ala Ser Val Val Cys Leu Leu Asn Asn Phe Tyr Pro Arg Glu Ala
        130                 135                 140


Lys Val Gln Trp Lys Val Asp Asn Ala Leu Gln Ser Gly Asn Ser Gln
145                 150                 155                 160

```
Glu Ser Val Thr Glu Gln Asp Ser Lys Asp Ser Thr Tyr Ser Leu Ser
            165                 170                 175


Ser Thr Leu Thr Leu Ser Lys Ala Asp Tyr Glu Lys His Lys Val Tyr
            180                 185                 190


Ala Cys Glu Val Thr His Gln Gly Leu Ser Ser Pro Val Thr Lys Ser
            195                 200                 205


Phe Asn Arg Gly Glu Cys
            210
```

<210> 2
<211> 214
<212> PRT
<213> Artificial Sequence

<220>
<223> Light chain of RAB4

<400> 2

```
Asp Ile Gln Met Thr Gln Ser Pro Gly Thr Leu Ser Leu Ser Pro Gly
1                   5                   10                  15


Glu Arg Ala Thr Leu Ser Cys Arg Ala Ser Gln Gly Val Ser Ser Ser
            20                  25                  30


Ser Leu Ala Trp Tyr Gln Gln Lys Pro Gly Gln Ala Pro Arg Leu Leu
            35                  40                  45


Ile Tyr Gly Thr Ser Ser Arg Ala Thr Gly Ile Pro Asp Arg Phe Ser
            50                  55                  60


Gly Ser Ala Ser Gly Thr Asp Phe Thr Leu Thr Ile Ser Arg Leu Gln
65                  70                  75                  80


Pro Glu Asp Phe Ala Val Tyr Tyr Cys Gln Gln Tyr Gly Arg Ser Leu
            85                  90                  95


Thr Phe Gly Gly Gly Thr Lys Val Glu Ile Lys Arg Thr Val Ala Ala
            100                 105                 110


Pro Ser Val Phe Ile Phe Pro Pro Ser Asp Glu Gln Leu Lys Ser Gly
            115                 120                 125


Thr Ala Ser Val Val Cys Leu Leu Asn Asn Phe Tyr Pro Arg Glu Ala
            130                 135                 140


Lys Val Gln Trp Lys Val Asp Asn Ala Leu Gln Ser Gly Asn Ser Gln
```

21

                    145                 150                 155                 160


Glu Ser Val Thr Glu Gln Asp Ser Lys Asp Ser Thr Tyr Ser Leu Ser
                165                 170                 175


Ser Thr Leu Thr Leu Ser Lys Ala Asp Tyr Glu Lys His Lys Val Tyr
            180                 185                 190


Ala Cys Glu Val Thr His Gln Gly Leu Ser Ser Pro Val Thr Lys Ser
            195                 200                 205


Phe Asn Arg Gly Glu Cys
        210


<210>   3
<211>   214
<212>   PRT
<213>   Artificial Sequence

<220>
<223>   Light chain of RAB0

<400>   3

Asp Ile Gln Met Thr Gln Ser Pro Gly Thr Leu Ser Leu Ser Pro Gly
1               5                   10                  15


Glu Arg Ala Thr Leu Ser Cys Arg Ala Ser Gln Gly Val Ser Ser Ser
            20                  25                  30


Ser Leu Ala Trp Tyr Gln Gln Lys Pro Gly Gln Ala Pro Arg Leu Leu
            35                  40                  45


Ile Tyr Gly Thr Ser Ser Arg Ala Thr Gly Ile Pro Asp Arg Phe Ser
        50                  55                  60


Gly Ser Ala Ser Gly Thr Asp Phe Thr Leu Thr Ile Ser Arg Leu Gln
65                  70                  75                  80


Pro Glu Asp Phe Ala Val Tyr Tyr Cys Gln Gln Tyr Gly Arg Ser Leu
                85                  90                  95


Thr Phe Gly Gly Gly Thr Lys Val Glu Ile Lys Arg Thr Val Ala Ala
            100                 105                 110


Pro Ser Val Phe Ile Phe Pro Pro Ser Asp Glu Gln Leu Lys Ser Gly
            115                 120                 125


Thr Ala Ser Val Val Cys Leu Leu Asn Asn Phe Tyr Pro Arg Glu Ala
        130                 135                 140

```
Lys Val Gln Trp Lys Val Asp Asn Ala Leu Gln Ser Gly Asn Ser Gln
145             150             155             160


Glu Ser Val Thr Glu Gln Asp Ser Lys Asp Ser Thr Tyr Ser Leu Ser
                165             170             175


Ser Thr Leu Thr Leu Ser Lys Ala Asp Tyr Glu Lys His Lys Val Tyr
            180             185             190


Ala Cys Glu Val Thr His Gln Gly Leu Ser Ser Pro Val Thr Lys Ser
        195             200             205


Phe Asn Arg Gly Glu Cys
    210


<210>   4
<211>   214
<212>   PRT
<213>   Artificial Sequence

<220>
<223>   Light chain of RAB2

<400>   4

Asp Ile Gln Met Thr Gln Ser Pro Val Thr Leu Ser Leu Ser Pro Gly
1               5               10              15


Glu Arg Ala Thr Leu Ser Cys Arg Ala Ser Gln Ser Val Arg Ser Ser
            20              25              30


Tyr Leu Ala Trp Tyr Gln Gln Lys Pro Gly Gln Thr Pro Arg Leu Leu
        35              40              45


Ile Tyr Gly Ala Ser Asn Arg Ala Thr Gly Ile Pro Asp Arg Phe Ser
    50              55              60


Gly Ser Gly Ser Gly Thr Asp Phe Thr Leu Thr Ile Ser Arg Leu Glu
65              70              75              80


Pro Glu Asp Phe Ala Val Tyr Tyr Cys Gln Gln Tyr Gly Asn Ser Leu
                85              90              95


Thr Phe Gly Gly Gly Thr Lys Val Glu Ile Lys Arg Thr Val Ala Ala
            100             105             110


Pro Ser Val Phe Ile Phe Pro Pro Ser Asp Glu Gln Leu Lys Ser Gly
            115             120             125
```

```
Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
        210


<210>  5
<211>  445
<212>  PRT
<213>  Artificial Sequence

<220>
<223>  Heavy chain of RAB9

<400>  5

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Ser Met Asn Trp Val Arg Gln Ala Pro Gly Lys Gly Leu Glu Trp Val
            35                  40                  45

Ser Ser Ile Gly Ser Ser Gly Gly Thr Thr Tyr Tyr Ala Asp Ser Val
        50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Gly Ser Gln Trp Leu Tyr Gly Met Asp Val Trp Gly Gln Gly Thr
            100                 105                 110
```

24

Thr Val Thr Val Ser Ser Ala Ser Thr Lys Gly Pro Ser Val Phe Pro
        115                 120                 125

Leu Ala Pro Cys Ser Arg Ser Thr Ser Glu Ser Thr Ala Ala Leu Gly
    130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Lys Thr Tyr Thr Cys Asn Val Asp His Lys Pro Ser
            195                 200                 205

Asn Thr Lys Val Asp Lys Arg Val Glu Ser Lys Tyr Gly Pro Pro Cys
    210                 215                 220

Pro Pro Cys Pro Ala Pro Glu Phe Leu Gly Gly Pro Ser Val Phe Leu
225                 230                 235                 240

Phe Pro Pro Lys Pro Lys Asp Thr Leu Met Ile Ser Arg Thr Pro Glu
            245                 250                 255

Val Thr Cys Val Val Val Asp Val Ser Gln Glu Asp Pro Glu Val Gln
            260                 265                 270

Phe Asn Trp Tyr Val Asp Gly Val Glu Val His Asn Ala Lys Thr Lys
        275                 280                 285

Pro Arg Glu Glu Gln Phe Asn Ser Thr Tyr Arg Val Val Ser Val Leu
    290                 295                 300

Thr Val Leu His Gln Asp Trp Leu Asn Gly Lys Glu Tyr Lys Cys Lys
305                 310                 315                 320

Val Ser Asn Lys Gly Leu Pro Ser Ser Ile Glu Lys Thr Ile Ser Lys
            325                 330                 335

Ala Lys Gly Gln Pro Arg Glu Pro Gln Val Tyr Thr Leu Pro Pro Ser
            340                 345                 350

Gln Glu Glu Met Thr Lys Asn Gln Val Ser Leu Thr Cys Leu Val Lys
            355                 360                 365

```
Gly Phe Tyr Pro Ser Asp Ile Ala Val Glu Trp Glu Ser Asn Gly Gln
    370             375             380

Pro Glu Asn Asn Tyr Lys Thr Thr Pro Pro Val Leu Asp Ser Asp Gly
385             390             395             400

Ser Phe Phe Leu Tyr Ser Arg Leu Thr Val Asp Lys Ser Arg Trp Gln
            405             410             415

Glu Gly Asn Val Phe Ser Cys Ser Val Met His Glu Ala Leu His Asn
            420             425             430

His Tyr Thr Gln Lys Ser Leu Ser Leu Ser Leu Gly Lys
        435             440             445
```

<210> 6
<211> 454
<212> PRT
<213> Artificial Sequence

<220>
<223> Heavy chain of RAB4

<400> 6

```
Glu Val Gln Leu Leu Glu Ser Gly Gly Gly Leu Val Gln Pro Gly Gly
1               5               10              15

Ser Leu Arg Leu Ser Cys Ala Ala Ser Gly Phe Thr Phe Ser Ile Tyr
            20              25              30

Ala Met Asp Trp Val Arg Gln Ala Pro Gly Lys Gly Leu Glu Trp Val
            35              40              45

Ser Gly Ile Val Pro Ser Gly Gly Phe Thr Lys Tyr Ala Asp Ser Val
            50              55              60

Lys Gly Arg Phe Thr Ile Ser Arg Asp Asn Ser Lys Asn Thr Leu Tyr
65              70              75              80

Leu Gln Met Asn Ser Leu Arg Ala Glu Asp Thr Ala Val Tyr Tyr Cys
                85              90              95

Ala Arg Val Asn Val Ile Ala Val Ala Gly Thr Gly Tyr Tyr Tyr Tyr
            100             105             110

Gly Met Asp Val Trp Gly Gln Gly Thr Thr Val Thr Val Ser Ser Ala
            115             120             125
```

26

Ser Thr Lys Gly Pro Ser Val Phe Pro Leu Ala Pro Cys Ser Arg Ser
    130                 135                 140

Thr Ser Glu Ser Thr Ala Ala Leu Gly Cys Leu Val Lys Asp Tyr Phe
    145                 150                 155                 160

Pro Glu Pro Val Thr Val Ser Trp Asn Ser Gly Ala Leu Thr Ser Gly
                165                 170                 175

Val His Thr Phe Pro Ala Val Leu Gln Ser Ser Gly Leu Tyr Ser Leu
            180                 185                 190

Ser Ser Val Val Thr Val Pro Ser Ser Ser Leu Gly Thr Lys Thr Tyr
        195                 200                 205

Thr Cys Asn Val Asp His Lys Pro Ser Asn Thr Lys Val Asp Lys Arg
    210                 215                 220

Val Glu Ser Lys Tyr Gly Pro Pro Cys Pro Pro Cys Pro Ala Pro Glu
225                 230                 235                 240

Phe Leu Gly Gly Pro Ser Val Phe Leu Phe Pro Pro Lys Pro Lys Asp
                245                 250                 255

Thr Leu Met Ile Ser Arg Thr Pro Glu Val Thr Cys Val Val Val Asp
            260                 265                 270

Val Ser Gln Glu Asp Pro Glu Val Gln Phe Asn Trp Tyr Val Asp Gly
        275                 280                 285

Val Glu Val His Asn Ala Lys Thr Lys Pro Arg Glu Glu Gln Phe Asn
    290                 295                 300

Ser Thr Tyr Arg Val Val Ser Val Leu Thr Val Leu His Gln Asp Trp
305                 310                 315                 320

Leu Asn Gly Lys Glu Tyr Lys Cys Lys Val Ser Asn Lys Gly Leu Pro
                325                 330                 335

Ser Ser Ile Glu Lys Thr Ile Ser Lys Ala Lys Gly Gln Pro Arg Glu
            340                 345                 350

Pro Gln Val Tyr Thr Leu Pro Pro Ser Gln Glu Glu Met Thr Lys Asn
        355                 360                 365

Gln Val Ser Leu Thr Cys Leu Val Lys Gly Phe Tyr Pro Ser Asp Ile
    370                 375                 380

Ala Val Glu Trp Glu Ser Asn Gly Gln Pro Glu Asn Asn Tyr Lys Thr
385                     390                 395                 400

Thr Pro Pro Val Leu Asp Ser Asp Gly Ser Phe Phe Leu Tyr Ser Arg
                405                 410                 415

Leu Thr Val Asp Lys Ser Arg Trp Gln Glu Gly Asn Val Phe Ser Cys
                420                 425                 430

Ser Val Met His Glu Ala Leu His Asn His Tyr Thr Gln Lys Ser Leu
                435                 440                 445

Ser Leu Ser Leu Gly Lys
        450


<210> 7
<211> 454
<212> PRT
<213> Artificial Sequence

<220>
<223> Heavy chain of RAB0

<400> 7

Glu Val Gln Leu Leu Glu Ser Gly Gly Gly Leu Val Gln Pro Gly Gly
1                   5                   10                  15

Ser Leu Arg Leu Ser Cys Ala Ala Ser Gly Phe Thr Phe Ser Trp Tyr
                20                  25                  30

Ala Met Asp Trp Val Arg Gln Ala Pro Gly Lys Gly Leu Glu Trp Val
                35                  40                  45

Ser Gly Ile Tyr Pro Ser Gly Gly Arg Thr Lys Tyr Ala Asp Ser Val
        50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Val Asn Val Ile Ala Val Ala Gly Thr Gly Tyr Tyr Tyr Tyr
                100                 105                 110

Gly Met Asp Val Trp Gly Gln Gly Thr Thr Val Thr Val Ser Ser Ala
                115                 120                 125

```
Ser Thr Lys Gly Pro Ser Val Phe Pro Leu Ala Pro Cys Ser Arg Ser
    130                 135                 140

Thr Ser Glu Ser Thr Ala Ala Leu Gly Cys Leu Val Lys Asp Tyr Phe
    145                 150                 155                 160

Pro Glu Pro Val Thr Val Ser Trp Asn Ser Gly Ala Leu Thr Ser Gly
                    165                 170                 175

Val His Thr Phe Pro Ala Val Leu Gln Ser Ser Gly Leu Tyr Ser Leu
                180                 185                 190

Ser Ser Val Val Thr Val Pro Ser Ser Ser Leu Gly Thr Lys Thr Tyr
                195                 200                 205

Thr Cys Asn Val Asp His Lys Pro Ser Asn Thr Lys Val Asp Lys Arg
    210                 215                 220

Val Glu Ser Lys Tyr Gly Pro Pro Cys Pro Pro Cys Pro Ala Pro Glu
225                 230                 235                 240

Phe Leu Gly Gly Pro Ser Val Phe Leu Phe Pro Pro Lys Pro Lys Asp
                245                 250                 255

Thr Leu Met Ile Ser Arg Thr Pro Glu Val Thr Cys Val Val Val Asp
                260                 265                 270

Val Ser Gln Glu Asp Pro Glu Val Gln Phe Asn Trp Tyr Val Asp Gly
                275                 280                 285

Val Glu Val His Asn Ala Lys Thr Lys Pro Arg Glu Glu Gln Phe Asn
    290                 295                 300

Ser Thr Tyr Arg Val Val Ser Val Leu Thr Val Leu His Gln Asp Trp
305                 310                 315                 320

Leu Asn Gly Lys Glu Tyr Lys Cys Lys Val Ser Asn Lys Gly Leu Pro
                325                 330                 335

Ser Ser Ile Glu Lys Thr Ile Ser Lys Ala Lys Gly Gln Pro Arg Glu
                340                 345                 350

Pro Gln Val Tyr Thr Leu Pro Pro Ser Gln Glu Glu Met Thr Lys Asn
                355                 360                 365

Gln Val Ser Leu Thr Cys Leu Val Lys Gly Phe Tyr Pro Ser Asp Ile
    370                 375                 380
```

```
Ala Val Glu Trp Glu Ser Asn Gly Gln Pro Glu Asn Asn Tyr Lys Thr
385             390             395                         400

Thr Pro Pro Val Leu Asp Ser Asp Gly Ser Phe Phe Leu Tyr Ser Arg
                405             410                 415

Leu Thr Val Asp Lys Ser Arg Trp Gln Glu Gly Asn Val Phe Ser Cys
            420             425                 430

Ser Val Met His Glu Ala Leu His Asn His Tyr Thr Gln Lys Ser Leu
            435             440                 445

Ser Leu Ser Leu Gly Lys
        450
```

<210> 8
<211> 454
<212> PRT
<213> Artificial Sequence

<220>
<223> Heavy chain of RAB2

<400> 8

```
Glu Val Gln Leu Leu Glu Ser Gly Gly Gly Leu Val Gln Pro Gly Gly
1               5               10                  15

Ser Leu Arg Leu Ser Cys Ala Ala Ser Gly Phe Thr Phe Ser Ile Tyr
            20              25                  30

Ala Met Asp Trp Val Arg Gln Ala Pro Gly Lys Gly Leu Glu Trp Val
        35              40                  45

Ser Gly Ile Val Pro Ser Gly Gly Phe Thr Lys Tyr Ala Asp Ser Val
    50              55                  60

Lys Gly Arg Phe Thr Ile Ser Arg Asp Asn Ser Lys Asn Thr Leu Tyr
65              70                  75                      80

Leu Gln Met Asn Ser Leu Arg Ala Glu Asp Thr Ala Val Tyr Tyr Cys
                85              90                  95

Ala Arg Val Asn Val Ile Ala Val Ala Gly Thr Gly Tyr Tyr Tyr Tyr
            100             105                 110

Gly Met Asp Val Trp Gly Gln Gly Thr Thr Val Thr Val Ser Ser Ala
            115             120                 125
```

```
Ser Thr Lys Gly Pro Ser Val Phe Pro Leu Ala Pro Cys Ser Arg Ser
    130             135             140

Thr Ser Glu Ser Thr Ala Ala Leu Gly Cys Leu Val Lys Asp Tyr Phe
    145             150             155             160

Pro Glu Pro Val Thr Val Ser Trp Asn Ser Gly Ala Leu Thr Ser Gly
                165             170             175

Val His Thr Phe Pro Ala Val Leu Gln Ser Ser Gly Leu Tyr Ser Leu
            180             185             190

Ser Ser Val Val Thr Val Pro Ser Ser Ser Leu Gly Thr Lys Thr Tyr
        195             200             205

Thr Cys Asn Val Asp His Lys Pro Ser Asn Thr Lys Val Asp Lys Arg
    210             215             220

Val Glu Ser Lys Tyr Gly Pro Pro Cys Pro Pro Cys Pro Ala Pro Glu
225             230             235             240

Phe Leu Gly Gly Pro Ser Val Phe Leu Phe Pro Pro Lys Pro Lys Asp
            245             250             255

Thr Leu Met Ile Ser Arg Thr Pro Glu Val Thr Cys Val Val Val Asp
        260             265             270

Val Ser Gln Glu Asp Pro Glu Val Gln Phe Asn Trp Tyr Val Asp Gly
        275             280             285

Val Glu Val His Asn Ala Lys Thr Lys Pro Arg Glu Glu Gln Phe Asn
    290             295             300

Ser Thr Tyr Arg Val Val Ser Val Leu Thr Val Leu His Gln Asp Trp
305             310             315             320

Leu Asn Gly Lys Glu Tyr Lys Cys Lys Val Ser Asn Lys Gly Leu Pro
            325             330             335

Ser Ser Ile Glu Lys Thr Ile Ser Lys Ala Lys Gly Gln Pro Arg Glu
        340             345             350

Pro Gln Val Tyr Thr Leu Pro Pro Ser Gln Glu Glu Met Thr Lys Asn
        355             360             365

Gln Val Ser Leu Thr Cys Leu Val Lys Gly Phe Tyr Pro Ser Asp Ile
    370             375             380
```

```
Ala Val Glu Trp Glu Ser Asn Gly Gln Pro Glu Asn Asn Tyr Lys Thr
385             390             395                 400

Thr Pro Pro Val Leu Asp Ser Asp Gly Ser Phe Phe Leu Tyr Ser Arg
                405             410                 415

Leu Thr Val Asp Lys Ser Arg Trp Gln Glu Gly Asn Val Phe Ser Cys
            420             425                 430

Ser Val Met His Glu Ala Leu His Asn His Tyr Thr Gln Lys Ser Leu
        435             440                 445

Ser Leu Ser Leu Gly Lys
    450


<210>  9
<211>  457
<212>  PRT
<213>  Artificial Sequence

<220>
<223>  RAM0hc

<400>  9

Glu Val Gln Leu Leu Glu Ser Gly Gly Gly Leu Val Gln Pro Gly Gly
1               5               10                  15

Ser Leu Arg Leu Ser Cys Ala Ala Ser Gly Phe Thr Phe Ser Trp Tyr
            20              25                  30

Ala Met Asp Trp Val Arg Gln Ala Pro Gly Lys Gly Leu Glu Trp Val
            35              40                  45

Ser Gly Ile Tyr Pro Ser Gly Gly Arg Thr Lys Tyr Ala Asp Ser Val
    50              55                  60

Lys Gly Arg Phe Thr Ile Ser Arg Asp Asn Ser Lys Asn Thr Leu Tyr
65              70              75                  80

Leu Gln Met Asn Ser Leu Arg Ala Glu Asp Thr Ala Val Tyr Tyr Cys
                85              90                  95

Ala Arg Val Asn Val Ile Ala Val Ala Gly Thr Gly Tyr Tyr Tyr Tyr
            100             105                 110

Gly Met Asp Val Trp Gly Gln Gly Thr Thr Val Thr Val Ser Ser Ala
        115             120                 125
```

```
Ser Thr Lys Gly Pro Ser Val Phe Pro Leu Ala Pro Ser Ser Lys Ser
    130             135             140

Thr Ser Gly Gly Thr Ala Ala Leu Gly Cys Leu Val Lys Asp Tyr Phe
    145             150             155             160

Pro Glu Pro Val Thr Val Ser Trp Asn Ser Gly Ala Leu Thr Ser Gly
                165             170             175

Val His Thr Phe Pro Ala Val Leu Gln Ser Ser Gly Leu Tyr Ser Leu
            180             185             190

Ser Ser Val Val Thr Val Pro Ser Ser Ser Leu Gly Thr Gln Thr Tyr
        195             200             205

Ile Cys Asn Val Asn His Lys Pro Ser Asn Thr Lys Val Asp Lys Arg
    210             215             220

Val Glu Pro Lys Ser Cys Asp Lys Thr His Thr Cys Pro Pro Cys Pro
225             230             235             240

Ala Pro Glu Leu Leu Gly Gly Pro Ser Val Phe Leu Phe Pro Pro Lys
                245             250             255

Pro Lys Asp Thr Leu Met Ile Ser Arg Thr Pro Glu Val Thr Cys Val
            260             265             270

Val Val Asp Val Ser His Glu Asp Pro Glu Val Lys Phe Asn Trp Tyr
        275             280             285

Val Asp Gly Val Glu Val His Asn Ala Lys Thr Lys Pro Arg Glu Glu
    290             295             300

Gln Tyr Asn Ser Thr Tyr Arg Val Val Ser Val Leu Thr Val Leu His
305             310             315             320

Gln Asp Trp Leu Asn Gly Lys Glu Tyr Lys Cys Lys Val Ser Asn Lys
            325             330             335

Ala Leu Pro Ala Pro Ile Glu Lys Thr Ile Ser Lys Ala Lys Gly Gln
            340             345             350

Pro Arg Glu Pro Gln Val Tyr Thr Leu Pro Pro Ser Arg Glu Glu Met
        355             360             365

Thr Lys Asn Gln Val Ser Leu Thr Cys Leu Val Lys Gly Phe Tyr Pro
    370             375             380
```

```
Ser Asp Ile Ala Val Glu Trp Glu Ser Asn Gly Gln Pro Glu Asn Asn
385             390             395             400

Tyr Lys Thr Thr Pro Pro Val Leu Asp Ser Asp Gly Ser Phe Phe Leu
                405             410             415

Tyr Ser Lys Leu Thr Val Asp Lys Ser Arg Trp Gln Gln Gly Asn Val
                420             425             430

Phe Ser Cys Ser Val Met His Glu Ala Leu His Asn His Tyr Thr Gln
                435             440             445

Lys Ser Leu Ser Leu Ser Pro Gly Lys
                450             455
```

<210> 10
<211> 214
<212> PRT
<213> Artificial Sequence

<220>
<223> RAM01c

<400> 10

```
Asp Ile Gln Met Thr Gln Ser Pro Gly Thr Leu Ser Leu Ser Pro Gly
1               5               10              15

Glu Arg Ala Thr Leu Ser Cys Arg Ala Ser Gln Gly Val Ser Ser Ser
                20              25              30

Ser Leu Ala Trp Tyr Gln Gln Lys Pro Gly Gln Ala Pro Arg Leu Leu
                35              40              45

Ile Tyr Gly Thr Ser Ser Arg Ala Thr Gly Ile Pro Asp Arg Phe Ser
                50              55              60

Gly Ser Ala Ser Gly Thr Asp Phe Thr Leu Thr Ile Ser Arg Leu Gln
65              70              75              80

Pro Glu Asp Phe Ala Val Tyr Tyr Cys Gln Gln Tyr Gly Arg Ser Leu
                85              90              95

Thr Phe Gly Gly Gly Thr Lys Val Glu Ile Lys Arg Thr Val Ala Ala
                100             105             110

Pro Ser Val Phe Ile Phe Pro Pro Ser Asp Glu Gln Leu Lys Ser Gly
                115             120             125
```

34

```
Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
    145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
                180                 185                 190

Ala Cys Glu Val Thr His Gln Gly Leu Ser Ser Pro Val Thr Lys Ser
            195                 200                 205

Phe Asn Arg Gly Glu Cys
        210
```

```
<210>  11
<211>  448
<212>  PRT
<213>  Artificial Sequence

<220>
<223>  RAM9hc

<400>  11
```

```
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ile Tyr
            20                  25                  30

Ser Met Asn Trp Val Arg Gln Ala Pro Gly Lys Gly Leu Glu Trp Val
        35                  40                  45

Ser Ser Ile Gly Ser Ser Gly Gly Thr Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Gly Ser Gln Trp Leu Tyr Gly Met Asp Val Trp Gly Gln Gly Thr
            100                 105                 110

Thr Val Thr Val Ser Ser Ala Ser Thr Lys Gly Pro Ser Val Phe Pro
```

                    115                          120                          125

Leu Ala Pro Ser Ser Lys Ser Thr Ser Gly Gly Thr Ala Ala Leu Gly
    130                 135                 140

Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Gln Thr Tyr Ile Cys Asn Val Asn His Lys Pro Ser
            195                 200                 205

Asn Thr Lys Val Asp Lys Arg Val Glu Pro Lys Ser Cys Asp Lys Thr
    210                 215                 220

His Thr Cys Pro Pro Cys Pro Ala Pro Glu Leu Leu Gly Gly Pro Ser
225                 230                 235                 240

Val Phe Leu Phe Pro Pro Lys Pro Lys Asp Thr Leu Met Ile Ser Arg
            245                 250                 255

Thr Pro Glu Val Thr Cys Val Val Val Asp Val Ser His Glu Asp Pro
            260                 265                 270

Glu Val Lys Phe Asn Trp Tyr Val Asp Gly Val Glu Val His Asn Ala
            275                 280                 285

Lys Thr Lys Pro Arg Glu Glu Gln Tyr Asn Ser Thr Tyr Arg Val Val
            290                 295                 300

Ser Val Leu Thr Val Leu His Gln Asp Trp Leu Asn Gly Lys Glu Tyr
305                 310                 315                 320

Lys Cys Lys Val Ser Asn Lys Ala Leu Pro Ala Pro Ile Glu Lys Thr
            325                 330                 335

Ile Ser Lys Ala Lys Gly Gln Pro Arg Glu Pro Gln Val Tyr Thr Leu
            340                 345                 350

Pro Pro Ser Arg Glu Glu Met Thr Lys Asn Gln Val Ser Leu Thr Cys
            355                 360                 365

```
Leu Val Lys Gly Phe Tyr Pro Ser Asp Ile Ala Val Glu Trp Glu Ser
    370             375             380

Asn Gly Gln Pro Glu Asn Asn Tyr Lys Thr Thr Pro Pro Val Leu Asp
    385             390             395             400

Ser Asp Gly Ser Phe Phe Leu Tyr Ser Lys Leu Thr Val Asp Lys Ser
                405             410             415

Arg Trp Gln Gln Gly Asn Val Phe Ser Cys Ser Val Met His Glu Ala
            420             425             430

Leu His Asn His Tyr Thr Gln Lys Ser Leu Ser Leu Ser Pro Gly Lys
        435             440             445


<210> 12
<211> 214
<212> PRT
<213> Artificial Sequence

<220>
<223> RAM91c

<400> 12

Asp Ile Gln Met Thr Gln Ser Pro Ser Ser Leu Ser Ala Ser Val Gly
1               5               10              15

Asp Arg Val Thr Ile Thr Cys Arg Ser Ser Gln Arg Ile Met Thr Tyr
            20              25              30

Leu Asn Trp Tyr Gln Gln Lys Pro Gly Lys Ala Pro Lys Leu Leu Ile
        35              40              45

Phe Val Ala Ser His Ser Gln Ser Gly Val Pro Ser Arg Phe Arg Gly
    50              55              60

Ser Gly Ser Glu Thr Asp Phe Thr Leu Thr Ile Ser Gly Leu Gln Pro
65              70              75              80

Glu Asp Ser Ala Thr Tyr Tyr Cys Gln Gln Ser Phe Trp Thr Pro Leu
                85              90              95

Thr Phe Gly Gly Gly Thr Lys Val Glu Ile Lys Arg Thr Val Ala Ala
            100             105             110

Pro Ser Val Phe Ile Phe Pro Pro Ser Asp Glu Gln Leu Lys Ser Gly
        115             120             125

Thr Ala Ser Val Val Cys Leu Leu Asn Asn Phe Tyr Pro Arg Glu Ala
```

                    130                    135                         140

Lys Val Gln Trp Lys Val Asp Asn Ala Leu Gln Ser Gly Asn Ser Gln
145                150                155                160

Glu Ser Val Thr Glu Gln Asp Ser Lys Asp Ser Thr Tyr Ser Leu Ser
                165                170                175

Ser Thr Leu Thr Leu Ser Lys Ala Asp Tyr Glu Lys His Lys Val Tyr
                180                185                190

Ala Cys Glu Val Thr His Gln Gly Leu Ser Ser Pro Val Thr Lys Ser
                195                200                205

Phe Asn Arg Gly Glu Cys
                210


<210> 13
<211> 457
<212> PRT
<213> Artificial Sequence

<220>
<223> RAM4hc

<400> 13

Glu Val Gln Leu Leu Glu Ser Gly Gly Gly Leu Val Gln Pro Gly Gly
1                5                10                15

Ser Leu Arg Leu Ser Cys Ala Ala Ser Gly Phe Thr Phe Ser Ile Tyr
                20                25                30

Ala Met Asp Trp Val Arg Gln Ala Pro Gly Lys Gly Leu Glu Trp Val
                35                40                45

Ser Gly Ile Val Pro Ser Gly Gly Phe Thr Lys Tyr Ala Asp Ser Val
                50                55                60

Lys Gly Arg Phe Thr Ile Ser Arg Asp Asn Ser Lys Asn Thr Leu Tyr
65                70                75                80

Leu Gln Met Asn Ser Leu Arg Ala Glu Asp Thr Ala Val Tyr Tyr Cys
                85                90                95

Ala Arg Val Asn Val Ile Ala Val Ala Gly Thr Gly Tyr Tyr Tyr Tyr
                100                105                110

Gly Met Asp Val Trp Gly Gln Gly Thr Thr Val Thr Val Ser Ser Ala
                115                120                125

```
Ser Thr Lys Gly Pro Ser Val Phe Pro Leu Ala Pro Ser Ser Lys Ser
    130             135             140

Thr Ser Gly Gly Thr Ala Ala Leu Gly Cys Leu Val Lys Asp Tyr Phe
    145             150             155             160

Pro Glu Pro Val Thr Val Ser Trp Asn Ser Gly Ala Leu Thr Ser Gly
                165             170             175

Val His Thr Phe Pro Ala Val Leu Gln Ser Ser Gly Leu Tyr Ser Leu
                180             185             190

Ser Ser Val Val Thr Val Pro Ser Ser Ser Leu Gly Thr Gln Thr Tyr
                195             200             205

Ile Cys Asn Val Asn His Lys Pro Ser Asn Thr Lys Val Asp Lys Arg
    210             215             220

Val Glu Pro Lys Ser Cys Asp Lys Thr His Thr Cys Pro Pro Cys Pro
225             230             235             240

Ala Pro Glu Leu Leu Gly Gly Pro Ser Val Phe Leu Phe Pro Pro Lys
                245             250             255

Pro Lys Asp Thr Leu Met Ile Ser Arg Thr Pro Glu Val Thr Cys Val
                260             265             270

Val Val Asp Val Ser His Glu Asp Pro Glu Val Lys Phe Asn Trp Tyr
                275             280             285

Val Asp Gly Val Glu Val His Asn Ala Lys Thr Lys Pro Arg Glu Glu
                290             295             300

Gln Tyr Asn Ser Thr Tyr Arg Val Val Ser Val Leu Thr Val Leu His
305             310             315             320

Gln Asp Trp Leu Asn Gly Lys Glu Tyr Lys Cys Lys Val Ser Asn Lys
                325             330             335

Ala Leu Pro Ala Pro Ile Glu Lys Thr Ile Ser Lys Ala Lys Gly Gln
                340             345             350

Pro Arg Glu Pro Gln Val Tyr Thr Leu Pro Pro Ser Arg Glu Glu Met
                355             360             365

Thr Lys Asn Gln Val Ser Leu Thr Cys Leu Val Lys Gly Phe Tyr Pro
```

370                     375                         380

Ser Asp Ile Ala Val Glu Trp Glu Ser Asn Gly Gln Pro Glu Asn Asn
385                 390             395                     400

Tyr Lys Thr Thr Pro Pro Val Leu Asp Ser Asp Gly Ser Phe Phe Leu
                405             410                 415

Tyr Ser Lys Leu Thr Val Asp Lys Ser Arg Trp Gln Gln Gly Asn Val
            420             425             430

Phe Ser Cys Ser Val Met His Glu Ala Leu His Asn His Tyr Thr Gln
            435             440             445

Lys Ser Leu Ser Leu Ser Pro Gly Lys
        450             455

<210> 14
<211> 214
<212> PRT
<213> Artificial Sequence

<220>
<223> RAM41c

<400> 14

Asp Ile Gln Met Thr Gln Ser Pro Gly Thr Leu Ser Leu Ser Pro Gly
1               5               10                  15

Glu Arg Ala Thr Leu Ser Cys Arg Ala Ser Gln Gly Val Ser Ser Ser
            20              25              30

Ser Leu Ala Trp Tyr Gln Gln Lys Pro Gly Gln Ala Pro Arg Leu Leu
        35              40              45

Ile Tyr Gly Thr Ser Ser Arg Ala Thr Gly Ile Pro Asp Arg Phe Ser
    50              55              60

Gly Ser Ala Ser Gly Thr Asp Phe Thr Leu Thr Ile Ser Arg Leu Gln
65              70              75                  80

Pro Glu Asp Phe Ala Val Tyr Tyr Cys Gln Gln Tyr Gly Arg Ser Leu
            85              90              95

Thr Phe Gly Gly Gly Thr Lys Val Glu Ile Lys Arg Thr Val Ala Ala
            100             105             110

Pro Ser Val Phe Ile Phe Pro Pro Ser Asp Glu Gln Leu Lys Ser Gly
        115             120             125

```
Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
                180                 185                 190

Ala Cys Glu Val Thr His Gln Gly Leu Ser Ser Pro Val Thr Lys Ser
    195                 200                 205

Phe Asn Arg Gly Glu Cys
    210
```

**Description**

The present invention pertains to anti-MIF antibodies, in particular their use in combination with cancer therapeutics, i.e. chemotherapeutics, in the treatment of cancer.

BACKGROUND

Macrophage migration inhibitory factor (MIF) is a cytokine initially isolated based upon its ability to inhibit the *in vitro* random migration of peritoneal exudate cells from tuberculin hypersensitive guinea pigs (containing macrophages) (Bloom et al. Science 1966,153,80-2; David et al. PNAS 1966, 56, 72-7). Today, MIF is known as a critical upstream regulator of the innate and acquired immune response that exerts a pleiotropic spectrum of activities.

The human MIF cDNA was cloned in 1989 (Weiser et al., PNAS 1989, 86, 7522-6), and its genomic localization was mapped to chromosome 22. The product of the human MIF gene is a protein with 114 amino acids (after cleavage of the N-terminal methionine) and an apparent molecular mass of about 12.5 kDa. MIF has no significant sequence homology to any other protein. The protein crystallizes as a trimer of identical subunits. Each monomer contains two antiparallel alpha-helices that pack against a four-stranded beta-sheet. The monomer has additional two beta-strands that interact with the beta-sheets of adjacent subunits to form the interface between monomers. The three subunits are arranged to form a barrel containing a solvent-accessible channel that runs through the center of the protein along a molecular three-fold axis (Sun et al. PNAS 1996, 93, 5191-5196).

It was reported that MIF secretion from macrophages was induced at very low concentrations of glucocorticoids (Calandra et al. Nature 1995, 377, 68-71). However, MIF also counter-regulates the effects of glucocorticoids and stimulates the secretion of other cytokines such as tumor necrosis factor TNF-$\alpha$ and interleukin IL-1ß (Baugh et al., Crit Care Med 2002, 30, S27-35). MIF was also shown e.g. to exhibit pro-angiogenic, pro-proliferative and anti-apoptotic properties, thereby promoting tumor cell growth (Mitchell, R.A., Cellular Signalling, 2004.16(1): p. 13-19; Lue, H. et al., Oncogene 2007.26(35): p. 5046-59). It is also e.g. directly associated with the growth of lymphoma, melanoma, and colon cancer (Nishihira et al. J Interferon Cytokine Res. 2000, 20:751-62).

MIF is a mediator of many pathologic conditions and thus associated with a variety of diseases including *inter alia* inflammatory bowel disease (IBD), rheumatoid arthritis (RA), acute respiratory distress syndrome (ARDS), asthma, glomerulonephritis, IgA nephropathy, myocardial infarction (MI), sepsis and cancer, though not limited thereto.

Polyclonal and monoclonal anti-MIF antibodies have been developed against recombinant human MIF (Shimizu et al., FEBS Lett. 1996; 381,199-202; Kawaguchi et al, Leukoc. Biol. 1986,39,223-232, and Weiser et al., Cell. Immunol. 1985, 90, 167-78).

Anti-MIF antibodies have been suggested for therapeutic use. Calandra et al., (J. Inflamm. (1995); 47, 39-51) reportedly used anti-MIF antibodies to protect animals from experimentally induced gram-negative and gram-positive septic shock. Anti-MIF antibodies were suggested as a means of therapy to modulate cytokine production in septic shock and other inflammatory disease states.

US 6,645,493 discloses monoclonal anti-MIF antibodies derived from hybridoma cells, which neutralize the biological activity of MIF. It could be shown in an animal model that these mouse-derived anti-MIF antibodies had a beneficial effect in the treatment of endotoxin-induced shock.

US 200310235584 discloses methods of preparing high affinity antibodies to MIF in animals in which the MIF gene has been homozygously knocked-out.

Glycosylation-inhibiting factor (GIF) is a protein described by Galat et al. (Eur. J. Biochem,1994, 224, 417-21). MIF and GIF are now recognized to be identical. Watarai et al. (PNAS 2000,97,13251-6) described polyclonal antibodies binding to different GIF epitopes to identify the biochemical nature of the posttranslational modification of GIF in Ts cells. Watarai et al, *supra,* reported that GIF occurs in different conformational isoforms *in vitro.* One type of isomer occurs by chemical modification of a single cysteine residue. The chemical modification leads to conformational changes within the GIF protein.

One of those medical diseases and disorders in which MIF has been implicated for the past decades is - as pointed out above - cancer. Cancer is generally treated via various routes, one of them being the use of so-called chemotherapeutic agents (which are the basis of anticancer chemotherapy). The concept underlying chemotherapy in the general sense thereof, posits that a disease or disorder (caused by bacteria, viruses, parasites and cancer cells) can be effectively treated by way of chemical compounds. One particular indication of chemotherapy is cancer. Chemotherapeutic agents can act for example by killing cells that divide more rapidly than other cells, and thus target cancer cells which commonly divide more rapidly than non-cancerous cells. Most chemotherapeutic agents drugs work by impairing cell division, i.e., they act at one or several stages of the cell cycle and thus are able to target cells that divide more rapidly. Chemotherapeutic agents can be either cytostatic, i.e. they slow down or abrogate the growth or division of cells; other chemotherapeutic drugs can cause damage to cells and kill them; in that case they are termed cytotoxic. Most cytotoxic drugs inflict a damage that *per se* does not suffice to kill a cell but that generates a stimulus to initiate programmed cell death (apoptosis).

In general, major classes of chemotherapeutic drugs are alkylating agents, anti-metabolites, anthracyclines, plant alkaloids, topoisomerases and other anti-tumour agents. Most commonly, as mentioned above, these drugs affect cell division; they can also affect DNA synthesis or function. Other chemotherapeutics do not directly interfere with DNA. These are newer classes of chemotherapeutic agents, which are referred to as signal interceptors, which include monoclonal antibodies and tyrosine kinase inhibitors like imatinib mesylate. Examples for alkylating agents, which alkylate nucleophilic functional groups are mechlorethamine, cyclophosphamide, chlorambucil, melphalane, trofosfamide, ifosfamide, carmustine, lomustine, dacarbazine, temozolomide, mitomycine C and many others. Cisplatin, carboplatin, oxaliplatin and other platinum containing compounds form stable complexes with DNA.

Cytotoxic anti-metabolites are folic acid analogues (e.g., methotrexat/aminopterin, raltitrexed, pemetrexed), purines (e.g., 6-mercaptopurine, azathioprine, thioguanine, fludarabine, cladribine) or pyrimidines (cytarabine, gemcitabine, 5-fuloruracil and its prodrugs, deazacytidine). Antimetabolites either inhibit DNA-synthesis by interfering with crucial steps in the de *novo* synthesis of purine and pyrimidine nucleotides or they become incorporated into DNA during the S-phase of the cell cycle, where they interfere with DNA-folding, DNA-repair or methylation. Alternatively, some compounds also become incorporated into RNA.

Examples for alkaloids and terpenoids which are derived from plants and block cell division by preventing microtubule function are vinca-alkaloids and taxanes. Particularly well known vinca-alkaloids are vincristine, vinblastine, vinorelbine and vindesine. Podophyllotoxin is an additional example of a plant-derived compound. An example for a taxane is docetaxel or paclitaxel. Estramustin is an example of a synthetic compound that targets tubulin.

Examples of topoisomerase inhibitors, which are inhibitors of enzymes that maintain the topology of DNA, include camphtotecines like irinotecan and topotecan (type 1 topoisomerase inhibitors) or amsacrin, etoposide, etoposide phosphate and teniposide (topoisomerase-type 2 inhibitors).

Finally, examples of antineoplastic intercalating agents include dactinomycin, doxorubicin, epirubicin, bleomycin and others.

A comprehensive overview is comprised in Goodman and Gilman, The Pharmacological Basis of Therapeutics, 12th Edition, "General Principles of Cancer Chemotherapy. Introduction" as shown below. Several tumors are susceptible to hormone therapy: glucocorticoids (e.g., prednisolone, dexamethasone and may others) promote apoptosis of lymphoma cells. They are therefore included in typical chemotherapeutic regimen. Similarly, several types of cancer are susceptible to hormonal interventions. This includes, e.g., breast cancer, ovarian cancer and prostate cancer. Hormonal ablation can be achieved by suppressing pituitary release of gonadotropins with gonadotropin-releasing hormone receptor agonists (e.g., buserelin, goserelin, leuprolide, hisrelin etc.), which induce desensitization of the receptor and hence inhibit hormone production, or with gonadotropin-releasing hormone receptor antagonists (e.g., degarelix). Alternatively, the action of estrogens and of androgens may be blocked by hormone receptor antagonists: compounds that act as partial agonists at estrogen receptors (also referred to as selective estrogen receptor modulators, SERM's) include tamoxifen, raloxifen and toremifen. Fulvestrant is an example of a pure estrogen recepor antagonist. Androgen receptors can be blocked by antagonists such as flutamide, bicalitamide and cyproterone. Finally, hormonal ablation can be achieved by blocking the pertinent enzymes, which are responsible for their synthesis. In the case of estrogens, it is the aromatase (CYP19), which is blocked by compounds such as aminoglutethimide, formestane, exemestane, anastrazole and letrozole. Androgen production can be suppressed by inhibiting the enzyme 17 $\alpha$-hydroxylase/C17,20 lyase (CYP17A1) with abiraterone. Regardless of by which approach hormonal input is blocked, the growth of susceptible cancer cells is suppressed and their apoptosis is promoted.

Although chemotherapeutic agents have been shown to be useful and successful in the treatment of several different cancer types, chemotherapeutic regimen have a range of side effects, depending on the type of medication used. Most common side effects include depression of the immune system which can result in potentially fatal infections, fatigue, anaemia, a tendency to bleed easily, gastrointestinal distress, like nausea and vomiting, diarrhoea or constipation and hair loss. Further, damage to specific organs may occur, which results e.g. in heart damage, liver damage, kidney damage, damage to the inner ear, damage to the peripheral nervous system and brain dysfunction.

All of these side effects increase severely if the dosage of the given chemotherapeutic drug is augmented. Decreasing the amount of the drug administered will usually also result in lower incidence and/or alleviated side effects.

In other cases it would be of benefit if the effects of the chemotherapeutic drugs could be enhanced or increased if high-dose treatment is necessary.

Thus, there remains an urgent need in the art for the provision of a therapy for cancer which allows reduction of the administration dose of a given chemotherapeutic and/or enhances the effects of a given chemotherapeutic.

## DESCRIPTION OF THE INVENTION

This object has been solved by the present invention.

In particular it could be shown that by a combination therapy of anti-MIF antibodies and a given chemotherapeutic agent a synergistic effect could be detected which would allow treatment of cancer with a lower dosage of the chemotherapeutic and/or achieving a higher effect with a similar dosage as compared to treating this cancer with the chemotherapeutic alone.

In particular, a treatment by a combination therapy of anti-oxMIF antibodies and a given chemotherapeutic could be shown to be associated with a synergistic effect, as described above and exemplified in the examples of the present invention.

Elevated MIF levels, i.e. levels of MIF in general are detected after the onset of various diseases, *inter alia* after the onset of cancer. However, MIF circulates also in healthy subjects, which makes a clear differentiation difficult. oxMIF, on the contrary, is not present in healthy subjects.

It has been discovered after thorough research of MIF and antibodies thereto that the antibodies RAB9, RAB4 and RAB0, as well as RAM9, RAM4 and RAM0, specifically bind to oxMIF (and are incapable of binding to redMIF).

In earlier experiments carried out by the inventors, it could be shown that oxidative procedures like cystine-mediated oxidation, GSSG (ox. Glutathione)-mediated oxidation or incubation of MIF with Proclin300 or protein crosslinkers (e.g. BMOE) causes binding of MIF to the above mentioned antibodies.

The surprising conclusions reached by the present inventors are:

- Redox modulation (Cys/Glu-mediated mild oxidation) of recombinant MIF (human, murine, rat, CHO, monkey)) or treatment of recombinant MIF with Proclin300 or protein crosslinkers leads to the binding of Baxter's anti-MIF antibodies RAB9, RAB4 and RAB0
- Reduction of oxMIF leads to the loss of Ab binding
- Specificity for oxMIF-isoforms correlates with biological Ab efficacy (*especially in vivo*).
- oxMIF levels can be correlated with a disease state.

This additional knowledge regarding (ox)MIF served as a basis for the further studies of the present inventors. Thus, preferred embodiments of the present invention are:

1. An anti-MIF antibody in combination with a chemotherapeutic agent for use in the treatment of cancer, wherein the chemotherapeutic agent is preferably gemcitabine, cisplatin, and/or doxorubicin.

2. The anti-MIF antibody in combination with chemotherapeutic agent according to item 1 for use in the treatment of cancer, wherein the cancer is selected from the following group: pancreatic cancer, ovarian cancer, prostate cancer, breast cancer, lung cancer and colon cancer, more preferred pancreatic cancer, prostate cancer and ovarian cancer.

3. The combination according to item 1 or 2 wherein the anti-MIF antibody is selected from the following group: anti-MIF antibody RAM9, RAM0 and/or RAM4.

4. The combination therapy according to any of items 1 to 3 wherein the chemotherapeutic is gemcitabine.

5. The combination therapy according to any of items 1 to 4, the anti-MIF antibody is RAM9, the chemotherapeutic is doxorubicin, optionally in combination with cisplatin and the cancer is ovarian cancer.

6. The combination therapy according to any of items 1 to 4, the anti-MIF antibody is RAM9, the chemotherapeutic is gemcitabine and the cancer is pancreas carcinoma.

7. The combination therapy according to any of items 1 to 4, the anti-MIF antibody is RAM0, the chemotherapeutic is doxorubicin, optionally in combination with cisplatin and the cancer is ovarian cancer.

8. The combination therapy according to any of items 1 to 4, the anti-MIF antibody is RAM0, the chemotherapeutic is gemcitabine and the cancer is pancreas carcinoma.

9. The combination therapy according to any of items 1 to 4, the anti-MIF antibody is RAM4, the chemotherapeutic is doxorubicin, optionally in combination with cisplatin and the cancer is ovarian cancer.

10. The combination therapy according to any of items 1 to 4, the anti-MIF antibody is RAM4, the chemotherapeutic is gemcitabine, and the cancer is pancreas carcinoma.

11. The combination therapy according to any of items 1 to 4, the anti-MIF antibody is RAM0, the chemotherapeutic is mitoxantrone, and the cancer is prostate cancer.

12. A kit comprising the combination as defined in any of items 1 - 11 above, and instructions for use.

13. A combination as defined in any of items 1 - 11 above, or the kit of item 12, for use in the treatment of cancer.

The above-mentioned antibodies are characterized and supported by both their sequences as well as by deposits as plasmids in *E.coli*, comprising either the light or the heavy chain of each of the above mentioned antibodies RAB0, RAB4 and RAB9, respectively, as well as RAM0, RAM4 and RAM9, respectively.

The plasmids are characterized by their DSM number which is the official number as obtained upon deposit under the Budapest Treaty with the German Collection of Microorganisms and Cell Cultures (DSMZ), Mascheroder Weg 1 b, Braunschweig, Germany. The plasmids were deposited in *E. coli* strains, respectively. The plasmid with the DSM 25110 number comprises the light chain sequence of the anti-MIF antibody RAB4. The plasmid with the DSM 25112 number comprises the heavy chain (IgG4) sequence of the anti-MIF antibody RAB4.

The co-expression of plasmids DSM 25110 and DSM 25112 in a suitable host cell results in the production of preferred anti-MIF antibody RAB4.

The plasmid with the DSM 25111 number comprises the light chain sequence of the anti-MIF antibody RAB9. The plasmid with the DSM 25113 number comprises the heavy chain (IgG4) sequence of the anti-MIF antibody RAB9.

The co-expression of plasmids DSM 25111 and DSM 25113 in a suitable host cell results in the production of preferred anti-MIF antibody RAB9.

The plasmid with the DSM 25114 number comprises the light chain sequence of the anti-MIF antibody RAB0. The plasmid with the DSM 25115 number comprises the heavy chain (IgG4) sequence of the anti-MIF antibody RAB0.

The co-expression of plasmids DSM 25114 and DSM 25115 in a suitable host cell results in the production of preferred anti-MIF antibody RAB0.

Similarly, the light and heavy chains of RAM0, RAM9 and RAM4 have been similarly deposited under the Budapest Treaty on April 12, 2012 with the DSMZ, Braunschweig, Germany. The following designations have been used:

RAM9 - heavy chain: E.coli GA.662-01.pRAM9hc - DSM 25860.
RAM4 - light chain: E.coli GA.906-04.pRAM4lc - DSM 25861.
RAM9 - light chain: E.coli GA.661-01.pRAM9lc - DSM 25859.
RAM4 - heavy chain: E.coli GA.657-02-pRAM4hc - DSM 25862.
RAM0 - light chain: E.coli GA.906-01.pRAM0lc - DSM 25863.
RAM0 - heavy chain: E.coli GA.784-01.pRAM0hc - DSM 25864.

The term "prophylactic" or "therapeutic" treatment is art-recognized and refers to administration of a drug to a subject. If it is administered prior to clinical manifestation of the unwanted condition (e.g., disease or other unwanted state of the subject) then the treatment is prophylactic, i.e., it protects the subject against developing the unwanted condition, whereas if administered after manifestation of the unwanted condition, the treatment is therapeutic (i.e., it is intended to diminish, ameliorate or maintain the existing unwanted condition or side effects therefrom).

As used herein an anti-(ox)MIF compound refers to any agent that attenuates, inhibits, opposes, counteracts, or decreases the biological activity of (ox)MIF. An anti(ox)MIF compound may be an agent that inhibits or neutralizes (ox)MIF activity, for example an antibody, particularly preferred, the antibodies as described herein, even more preferred the antibodies RAB9, RAB4 and/or RAB0. Very preferred antibodies are RAM9, RAM4 and/or RAM0.

The preferred MIF antagonist in accordance with the present invention is an anti-MIF antibody. Even more preferred the anti-MIF antibody is an antibody against oxMIF. In other embodiments, the anti-oxMIF antibodies, e.g., the antibodies mentioned above or an antigen-binding portion thereof bind oxMIF with a $K_D$ of less than 100 nM, preferably a $K_D$ of less than 50 nM, even more preferred with a $K_D$ of less than 10nM. Very preferred, the antibodies bind to oxMIF with a $K_D$ of less than 5 nM.

The invention further relates to kits comprising an anti-MIF antibody or an antigen-binding portion thereof as well as a chemotherapeutic agent according to the invention. A kit may include in addition to the antibody and the chemotherapeutic agent, further therapeutic agents and uses thereof. A kit also can include instructions for use in a therapeutic method.

Earlier results have shown that an anti-MIF antibody that only binds oxMIF and does not bind redMIF and further inhibits GCO and/or cell proliferation induces a beneficial effect in an animal model.

## DETAILED DESCRIPTION OF THE INVENTION

The invention is further described in the figures as enclosed.

## Description of the figures:

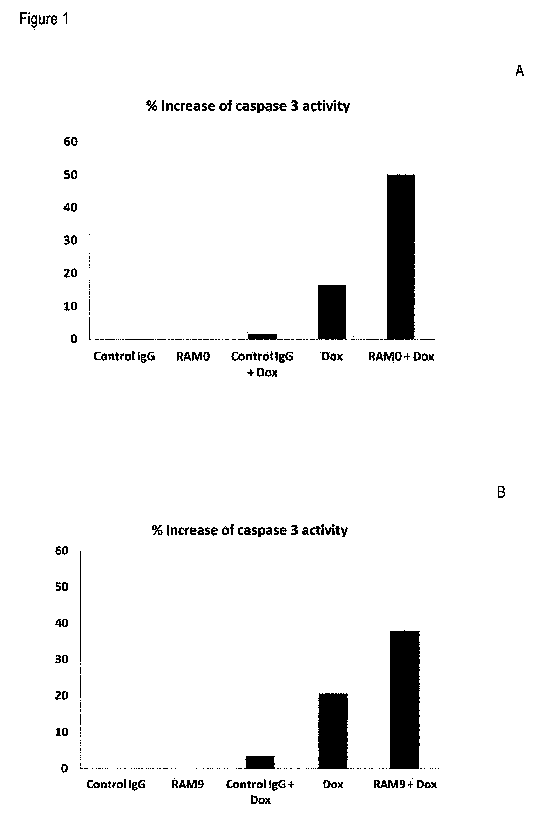

Figure 1: Elevation of Caspase 3 levels when A2780adr ovarian cancer cells are treated with doxorubicin in combination with RAM0 (A) and RAM9 (B). The percentage increased caspase 3 activity over non-treated cells is indicated. Cells were treated with a human isotype control IgG (Control IgG), anti-MIF antibody RAM0 or RAM9 alone, a combination of control IgG and doxorubicin (Control IgG + Dox), doxorubicin alone (Dox) or a combination of anti-MIF antibody and doxorubicin (RAM0+Dox or RAM9+Dox).

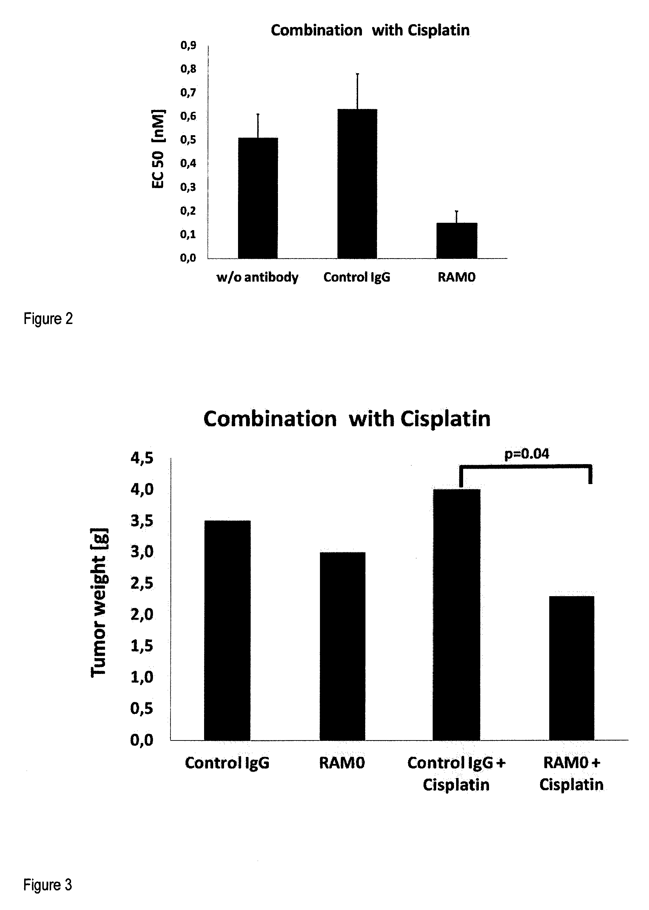

Figure 2: *In vitro* combination of RAM0 and cisplatin in a cisplatin-dependent cell killing assay using the human ovarian cancer cell line A2780. The EC50 of cisplatin in absence of antibody (w/o antibody) or in the presence of a human isotype control IgG or anti-MIF antibody RAM0 is depicted. Data are the means ± standard deviation of 9 independent experiments.

**Figure 3:** *In vivo* combination of RAM0 and cisplatin in a mouse xenograft model using the human ovarian cancer cell line A2780. Tumor weights are indicated after treatment of inoculated mice with a human control IgG or with RAM0. Antibodies were applied either alone or in combination with cisplatin.

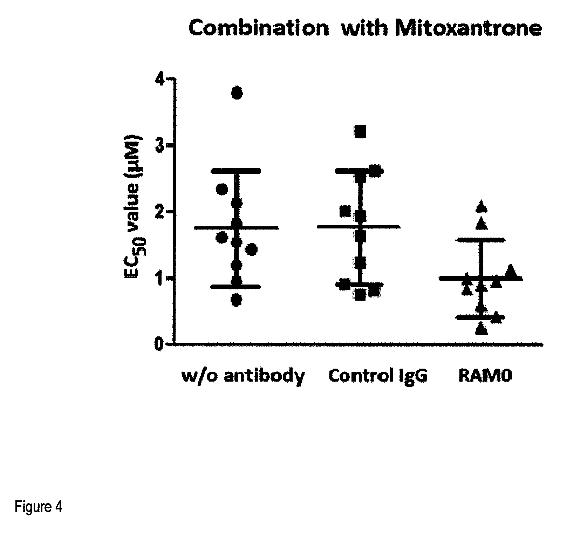

**Figure 4:** *In vitro* combination of RAM0 and mitoxantrone in a mitoxantrone-dependent cell killing assay using the human prostate cancer cell line LnCAP. The EC50s of mixoantrone in absence of antibody (w/o antibody) or in the presence of a human isotype control IgG or anti-MIF antibody RAM0 are depicted. The single values as well as the mean ± standard deviation of 10 independent experiments are shown.

**Definitions and General Techniques**

Unless otherwise defined herein, scientific and technical terms used in connection with the present invention shall have the meanings that are commonly understood by those of ordinary skill in the art. Generally, nomenclatures used in connection with, and techniques of, cell and tissue culture, molecular biology, immunology, microbiology, genetics and protein and nucleic acid chemistry described herein are those well known and commonly used in the art. The methods and techniques of the present invention are generally performed according to conventional methods well known in the art and as described in various general and more specific references that are cited and discussed throughout the present specification unless otherwise indicated. See, e.g., Sambrook et al., Molecular Cloning: A Laboratory Manual, 2nd ed., Cold Spring Harbor Laboratory Press, Cold Spring Harbor, N.Y. (1989) and Ausubel et al., Current Protocols in Molecular Biology, Greene Publishing Associates (1992), and Harlow and Lane Antibodies: A Laboratory Manual, Cold Spring Harbor Laboratory Press, Cold Spring Harbor, N.Y. (1990), which are incorporated herein by reference. "MIF" or "macrophage migration inhibitory factor" refers to the protein, which is known as a critical mediator in the immune and inflammatory response, and as a counterregulator of glucocorticoids. MIF includes mammalian MIF, specifically human MIF (Swiss-Prot primary accession number: P14174), wherein the monomeric form is encoded as a 115 amino acid protein but is produced as a 114 amino acid protein due to cleavage of the initial methionine. "MIF" also includes "GIF" (glycosylation-inhibiting factor) and other forms of MIF such as fusion proteins of MIF. The numbering of the amino acids of MIF starts with the N-terminal methionine (amino acid 1) and ends with the C-terminal alanine (amino acid 115). "oxidized MIF" or oxMIF is defined for the purposes of the invention as an isoform of MIF that occurs by treatment of MIF with mild oxidizing reagents, such as Cystine. As has been shown by the present inventors, recombinant oxMIF that has been treated this way comprises isoform(s) of MIF that share structural rearrangements with oxMIF that (e.g.) occurs *in vivo* after challenge of animals with bacteria.

redMIF is defined for the purposes of this invention as reduced MIF and is MIF which does not bind to RAB0, RAB9 and/or RAB4.

The anti-oxMIF antibodies described in this invention are able to discriminate between ox and red MIF, which are generated by mild oxidation or reduction, respectively. The anti-oxMIF antibodies are useful to specifically detect oxMIF. Discrimination between these conformers is assessed by ELISA or surface plasmon resonance. Both techniques can be performed as well known to a person skilled in the art and as described below.

**Assessing differential binding of the antibodies by Biacore.**

Binding kinetics of oxMIF and redMIF to antibody RAB9 and RAB0 are examined by surface plasmon resonance analysis using a Biacore 3000 System. The antibodies were coated on a CM5 (= carboxymethylated dextran) chip and recombinant MIF protein, pre-incubated with 0.2% Proclin300, were injected. (Proclin300 consists of oxidative isothiazolones that stabilize the oxMIF structure). In native HBS-EP buffer (= Biacore running buffer) without addition of ProClin300, none of the recombinant MIF proteins bound to RAB9, RAB0 or to the reference antibody (irrelevant isotype control antibody) used as negative (background) binding control.

In a preferred embodiment, oxMIF is MIF which is differentially bound by antibody RAB9, RAB4 and/or RAB0 or an antigen-binding fragment thereof, meaning that these antibodies do bind to oxMIF while redMIF is not bound by either one of these antibodies.

In other embodiments, the anti-oxMIF antibodies, e.g., the antibodies mentioned above or an antigen-binding portion thereof bind oxMIF with a $K_D$ of less than 100 nM, preferably a $K_D$ of less than 50 nM, even more preferred with a $K_D$ of less than 10 nM. Particularly preferred, the antibodies of the invention bind to oxMIF with a $K_D$ of less than 5 nM.

The antibodies of the invention, as defined hereinabove and hereinafter, have the same specificities. They thus also show similar results in the experiments as carried out by the present inventors.

(Non-)binding of an antibody, e.g. RAB9, RAB4 or RAB0 or RAM9, RAM4 or RAM0 (to oxMIF or redMIF) can be determined as generally known to a person skilled in the art, examples being any one of the following methods: ELISA with recombinant MIF in its reduced or oxidized state, or surface plasmon resonance using recombinant MIF in its reduced or oxidized state, like the well known Biacore assay, described above.

A preferred method for the determination of binding is surface plasmon resonance of an antibody to e.g. rec. (ox)MIF whereupon "binding" is meant to be represented by a $K_D$ of less than 100 nM preferably less than 50 nM, even more preferred less than 10 nM whereas the non-binding to redMIF is characterized by a $K_D$ of more than 400 nM. "Binding" and "specific binding" is used interchangeably here to denote the above. "Differential binding" in the context of this application means that a compound, in particular the antibodies as described herein, bind to oxMIF (e.g., with the $K_D$ values mentioned above) while they do not bind to redMIF (with non-binding again being defined as above).

An "antibody" refers to an intact antibody or an antigen-binding portion that competes with the intact antibody for (specific) binding. See generally, Fundamental Immunology, Ch. 7 (Paul, W., ed., 2nd ed. Raven Press, N.Y. (1989)) (incorporated by reference). The term antibody includes human antibodies, mammalian antibodies, isolated antibodies and genetically engineered forms such as chimeric, camelide/camelized or humanized antibodies, though not being limited thereto.

The term "antigen-binding portion" of an antibody refers to one or more fragments of an antibody that retain the ability to specifically bind to an antigen (e.g., (ox)MIF). Antigen-binding portions may be produced by recombinant DNA techniques or by enzymatic or chemical cleavage of intact antibodies. Antigen-binding portions include e.g. - though not limited thereto - the following: Fab, Fab', F(ab')2, Fv, and complementarity determining region (CDR) fragments, single-chain antibodies (scFv), chimeric antibodies, antibodies and polypeptides that contain at least a portion of an antibody that is sufficient to confer specific antigen binding to the polypeptide, i.e., ox or redMIF. From N-terminus to C-terminus, both the mature light and heavy chain variable domains comprise the regions FR1, CDR1, FR2, CDR2, FR3, CDR3 and FR4. The assignment of amino acids to each domain is in accordance with the definitions of Kabat, Sequences of Proteins of Immunological Interest (National Institutes of Health, Bethesda, Md. (1987 and 1991)), Chothia et al. J. Mol. Biol. 196:901-917 (1987), or Chothia et al., Nature 342:878-883 (1989). An antibody or antigen-binding portion thereof can be derivatized or linked to another functional molecule (e.g., another peptide or protein). For example, an antibody or antigen- binding portion thereof can be functionally linked to one or more other molecular entities, such as another antibody (e.g., a bispecific antibody or a diabody), a detectable agent, a cytotoxic agent, a pharmaceutical agent, and/or a linking molecule.

The term "KD" refers here, in accordance with the general knowledge of a person skilled in the art to the equilibrium dissociation constant of a particular antibody with the respective antigen. This equilibrium dissociation constant measures the affinity. The affinity determines how much complex is formed at equilibrium (steady state where association balances dissociation) (here: ox or redMIF and antibody).

$$ka = \text{association rate constant } [M\text{-}1\ s\text{-}1]$$

$$kd = \text{dissociation rate constant } [s\text{-}1]$$

$$KD = \text{equilibrium dissociation constant} = kd/ka\ [M]$$

The term "human antibody" refers to any antibody in which the variable and constant domains are human sequences. The term encompasses antibodies with sequences derived from human genes, but which have been changed, e.g. to decrease possible immunogenicity, increase affinity, eliminate cysteines that might cause undesirable folding, etc. The term encompasses such antibodies produced recombinantly in non-human cells, which might e.g. impart glycosylation not typical of human cells.

The term "humanized antibody" refers to antibodies comprising human sequences and containing also non-human sequences; in particular, a "humanized antibody" refers to a non-human antibody where human sequences have been added and/or replace the non-human sequences.

The term "camelized antibody" refers to antibodies wherein the antibody structure or sequences has been changed to more closely resemble antibodies from camels, also designated camelid antibodies. Methods for the design and production of camelized antibodies are part of the general knowledge of a person skilled in the art.

The term "chimeric antibody" refers to an antibody that comprises regions from two or more different species. The term "isolated antibody" or "isolated antigen-binding portion thereof' refers to an antibody or an antigen-binding portion thereof that has been identified and selected from an antibody source such as a phage display library or a B-cell repertoire.

The production of the anti-(ox)MIF antibodies according to the present invention includes any method for the generation of recombinant DNA by genetic engineering, e.g. via reverse transcription of RNA and/or amplification of DNA and cloning into expression vectors. In some embodiments, the vector is a viral vector, wherein additional DNA segments may be ligated into the viral genome. In some embodiments, the vector is capable of autonomous replication in a host cell into which it is introduced (e.g., bacterial vectors having a bacterial origin of replication and episomal mammalian vectors). In other embodiments, the vector (e.g., non-episomal mammalian vectors) can be integrated into the genome of a host cell upon introduction into the host cell, and thereby replicated along with the host genome. Moreover, certain vectors are capable of directing the expression of genes to which they are operatively linked. Such vectors are referred to herein as "recombinant expression vectors" (or simply, "expression vectors").

Anti-(ox)MIF antibodies can be produced *inter alia* by means of conventional expression vectors, such as bacterial vectors (e.g., pBR322 and its derivatives), or eukaryotic vectors. Those sequences that encode the antibody can be provided with regulatory sequences that regulate the replication, expression and/or secretion from the host cell. These regulatory sequences comprise, for instance, promoters (e.g., CMV or SV40) and signal sequences. The expression vectors can also comprise selection and amplification markers, such as the dihydrofolate reductase gene (DHFR), hygromycin-B-phosphotransferase, and thymidine-kinase. The components of the vectors used, such as selection markers, replicons, enhancers, can either be commercially obtained or prepared by means of conventional methods. The vectors can be constructed for the expression in various cell cultures, e.g., in mammalian cells such as CHO, COS, HEK293, NSO, fibroblasts, insect cells, yeast or bacteria such as *E.coli.* In some instances, cells are used that allow for optimal glycosylation of the expressed protein.

The anti-(ox)MIF antibody light chain gene(s) and the anti-(ox)MIF antibody heavy chain gene(s) can be inserted into separate vectors or the genes are inserted into the same expression vector. The antibody genes are inserted into the expression vector by standard methods, e.g., ligation of complementary restriction sites on the antibody gene fragment and vector, or blunt end ligation if no restriction sites are present.

The production of anti-(ox)MIF antibodies or antigen-binding fragments thereof may include any method known in the art for the introduction of recombinant DNA into eukaryotic cells by transfection, e.g. via electroporation or microinjection. For example, the recombinant expression of anti-(ox)MIF antibody can be achieved by introducing an expression plasmid containing the anti-(ox)MIF antibody encoding DNA sequence under the control of one or more regulating sequences such as a strong promoter, into a suitable host cell line, by an appropriate transfection method resulting in cells having the introduced sequences stably integrated into the genome. The lipofection method is an example of a transfection method which may be used according to the present invention.

The production of anti-(ox)MIF antibodies may also include any method known in the art for the cultivation of said transformed cells, e.g. in a continuous or batchwise manner, and the expression of the anti-(ox)MIF antibody, e.g. constitutive or upon induction. It is referred in particular to WO 2009/086920 for further reference for the production of anti-(ox)MIF antibodies. In a preferred embodiment, the anti-(ox)MIF antibodies as produced according to the present invention bind to oxMIF or an epitope thereof. Particularly preferred antibodies in accordance with the present invention are antibodies RAB9, RAB4 and/or RAB0, as well as RAM9, RAM4 and/or RAM0.

The sequences of these antibodies are partly also disclosed in WO 2009/086920; see in addition the sequence list of the present application and the following:

SEQ ID NO: 1 for the amino acid sequence of the light chain of RAB9:

DIQMTQSPSS LSASVGDRVT ITCRSSQRIM TYLNWYQQKP GKAPKLLIFV ASHSQSGVPS RFRGSGSETD FTLTISGLQP EDSATYYCQQ SFWTPLTFGG GTKVEIKRTV AAPSVFIFPP SDEQLKSGTA SVVCLLNNFY PREAKVQWKV DNALQSGNSQ ESVTEQDSKD STYSLSSTLT LSKADYEKHK VYACEVTHQG LSSPVTKSFN RGEC,

SEQ ID NO: 2 for the amino acid sequence of the light chain of RAB4:

DIQMTQSPGT LSLSPGERAT LSCRASQGVS SSSLAWYQQK PGQAPRLLIY GTSSRATGIP DRFSGSASGT DFTLTISRLQ PEDFAVYYCQ QYGRSLTFGG GTKVEIKRTV AAPSVFIFPP SDEQLKSGTA SVVCLLNNFY PREAKVQWKV DNALQSGNSQ ESVTEQDSKD STYSLSSTLT LSKADYEKHK VYACEVTHQG LSSPVTKSFN RGEC,

SEQ ID NO: 3 for the amino acid sequence of the light chain of RAB0:

DIQMTQSPGT LSLSPGERAT LSCRASQGVS SSSLAWYQQK PGQAPRLLIY GTSSRATGIP DRFSGSASGT DFTLTISRLQ PEDFAVYYCQ QYGRSLTFGG GTKVEIKRTV AAPSVFIFPP SDEQLKSGTA SVVCLLNNFY PREAKVQWKV DNALQSGNSQ ESVTEQDSKD STYSLSSTLT LSKADYEKHK VYACEVTHQG LSSPVTKSFN RGEC,

SEQ ID NO: 4 for the amino acid sequence of the light chain of RAB2:

DIQMTQSPVT LSLSPGERAT LSCRASQSVR SSYLAWYQQK PGQTPRLLIY GASNRATGIP DRFSGSGSGT DFTLTISRLE PEDFAVYYCQ QYGNSLTFGG GTKVEIK^RTV AAPSVFIFPP SDEQLKSGTA SVVCLLNNFY PREAKVQWKV DNALQSGNSQ ESVTEQDSKD STYSLSSTLT LSKADYEKHK VYACEVTHQG LSSPVTKSFN RGEC,

SEQ ID NO: 5 for the amino acid sequence of the heavy chain of RAB9:

EVQLLESGGG LVQPGGSLRL SCAASGFTFS IYSMNWVRQA PGKGLEWVSS
IGSSGGTTYY ADSVKGRFTI SRDNSKNTLY LQMNSLRAED TAVYYCAGSQ
WLYGMDVWGQ GTTVTVSSAS TKGPSVFPLA PCSRSTSEST AALGCLVKDY
FPEPVTVSWN SGALTSGVHT FPAVLQSSGL YSLSSVVTVP SSSLGTKTYT
CNVDHKPSNT KVDKRVESKY GPPCPPCPAP EFLGGPSVFL FPPKPKDTLM
ISRTPEVTCV VVDVSQEDPE VQFNWYVDGV EVHNAKTKPR EEQFNSTYRV

VSVLTVLHQD WLNGKEYKCK VSNKGLPSSI EKTISKAKGQ PREPQVYTLP
PSQEEMTKNQ VSLTCLVKGF YPSDIAVEWE SNGQPENNYK TTPPVLDSDG
SFFLYSRLTV DKSRWQEGNV FSCSVMHEAL HNHYTQKSLS LSLGK,

SEQ ID NO: 6 for the amino acid sequence of the heavy chain of RAB4:

EVQLLESGGG LVQPGGSLRL SCAASGFTFS IYAMDWVRQA PGKGLEWVSG
IVPSGGFTKY ADSVKGRFTI SRDNSKNTLY LQMNSLRAED TAVYYCARVN
VIAVAGTGYY YYGMDVWGQG TTVTVSSAST KGPSVFPLAP CSRSTSESTA
ALGCLVKDYF PEPVTVSWNS GALTSGVHTF PAVLQSSGLY SLSSVVTVPS
SSLGTKTYTC NVDHKPSNTK VDKRVESKYG PPCPPCPAPE FLGGPSVFLF
PPKPKDTLMI SRTPEVTCVV VDVSQEDPEV QFNWYVDGVE VHNAKTKPRE
EQFNSTYRVV SVLTVLHQDW LNGKEYKCKV SNKGLPSSIE KTISKAKGQP
REPQVYTLPP SQEEMTKNQV SLTCLVKGFY PSDIAVEWES NGQPENNYKT
TPPVLDSDGS FFLYSRLTVD KSRWQEGNVF SCSVMHEALH NHYTQKSLSL
SLGK,

SEQ ID NO: 7 for the amino acid sequence of the heavy chain of RAB0:

9

EVQLLESGGG LVQPGGSLRL SCAASGFTFS WYAMDWVRQA PGKGLEWVSG IYPSGGRTKY ADSVKGRFTI SRDNSKNTLY LQMNSLRAED TAVYYCARVN VIAVAGTGYY YYGMDVWGQG TTVTVSSAST KGPSVFPLAP CSRSTSESTA ALGCLVKDYF PEPVTVSWNS GALTSGVHTF PAVLQSSGLY SLSSVVTVPS SSLGTKTYTC NVDHKPSNTK VDKRVESKYG PPCPPCPAPE FLGGPSVFLF PPKPKDTLMI SRTPEVTCVV VDVSQEDPEV QFNWYVDGVE VHNAKTKPRE EQFNSTYRVV SVLTVLHQDW LNGKEYKCKV SNKGLPSSIE KTISKAKGQP REPQVYTLPP SQEEMTKNQV SLTCLVKGFY PSDIAVEWES NGQPENNYKT TPPVLDSDGS FFLYSRLTVD KSRWQEGNVF SCSVMHEALH NHYTQKSLSL SLGK,

SEQ ID NO: 8 for the amino acid sequence of the heavy chain of RAB2:

EVQLLESGGG LVQPGGSLRL SCAASGFTFS IYAMDWVRQA PGKGLEWVSG IVPSGGFTKY ADSVKGRFTI SRDNSKNTLY LQMNSLRAED TAVYYCARVN VIAVAGTGYY YYGMDVWGQG TTVTVSSAST KGPSVFPLAP CSRSTSESTA ALGCLVKDYF PEPVTVSWNS GALTSGVHTF PAVLQSSGLY SLSSVVTVPS SSLGTKTYTC NVDHKPSNTK VDKRVESKYG PPCPPCPAPE FLGGPSVFLF PPKPKDTLMI SRTPEVTCVV VDVSQEDPEV QFNWYVDGVE VHNAKTKPRE EQFNSTYRVV SVLTVLHQDW LNGKEYKCKV SNKGLPSSIE KTISKAKGQP REPQVYTLPP SQEEMTKNQV SLTCLVKGFY PSDIAVEWES NGQPENNYKT TPPVLDSDGS FFLYSRLTVD KSRWQEGNVF SCSVMHEALH NHYTQKSLSL SLGK,

SEQ ID NO: 9 for the amino acid sequence of RAM0hc:

EVQLLESGGG LVQPGGSLRL SCAASGFTFS WYAMDWVRQA PGKGLEWVSG IYPSGGRTKY ADSVKGRFTI SRDNSKNTLY LQMNSLRAED TAVYYCARVN VIAVAGTGYY YYGMDVWGQG TTVTVSSAST KGPSVFPLAP SSKSTSGGTA ALGCLVKDYF PEPVTVSWNS GALTSGVHTF PAVLQSSGLY SLSSVVTVPS SSLGTQTYIC NVNHKPSNTK VDKRVEPKSC DKTHTCPPCP APELLGGPSV FLFPPKPKDT LMISRTPEVT CVVVDVSHED PEVKFNWYVD GVEVHNAKTK PREEQYNSTY RVVSVLTVLH QDWLNGKEYK CKVSNKALPA PIEKTISKAK GQPREPQVYT LPPSREEMTK NQVSLTCLVK GFYPSDIAVE WESNGQPENN YKTTPPVLDS DGSFFLYSKL TVDKSRWQQG NVFSCSVMHE ALHNHYTQKS LSLSPGK,

SEQ ID NO: 10 for the amino acid sequence of RAM0lc:

DIQMTQSPGT LSLSPGERAT LSCRASQGVS SSSLAWYQQK PGQAPRLLIY GTSSRATGIP DRFSGSASGT
DFTLTISRLQ PEDFAVYYCQ QYGRSLTFGG GTKVEIKRTV AAPSVFIFPP SDEQLKSGTA SVVCLLNNFY
PREAKVQWKV DNALQSGNSQ ESVTEQDSKD STYSLSSTLT LSKADYEKHK VYACEVTHQG
LSSPVTKSFN RGEC,

SEQ ID NO: 11 for the amino acid sequence of RAM9hc:

EVQLLESGGG LVQPGGSLRL SCAASGFTFS IYSMNWVRQA PGKGLEWVSS IGSSGGTYY
ADSVKGRFTI SRDNSKNTLY LQMNSLRAED TAVYYCAGSQ WLYGMDVWGQ GTTVTVSSAS
TKGPSVFPLA PSSKSTSGGT AALGCLVKDY FPEPVTVSWN SGALTSGVHT FPAVLQSSGL YSLSSVVTVP
SSSLGTQTYI
CNVNHKPSNT KVDKRVEPKS CDKTHTCPPC PAPELLGGPS VFLFPPKPKD TLMISRTPEV TCVVVDVSHE
DPEVKFNWYV DGVEVHNAKT KPREEQYNST YRVVSVLTVL HQDWLNGKEY KCKVSNKALP APIEKTISKA
KGQPREPQVY TLPPSREEMT KNQVSLTCLV KGFYPSDIAV EWESNGQPEN NYKTTPPVLD
SDGSFFLYSK LTVDKSRWQQ GNVFSCSVMH EALHNHYTQK SLSLSPGK,

SEQ ID NO: 12 for the amino acid sequence of RAM9lc:

DIQMTQSPSS LSASVGDRVT ITCRSSQRIM TYLNWYQQKP GKAPKLLIFV ASHSQSGVPS RFRGSGSETD
FTLTISGLQP EDSATYYCQQ SFWTPLTFGG GTKVEIKRTV AAPSVFIFPP SDEQLKSGTA SVVCLLNNFY
PREAKVQWKV DNALQSGNSQ ESVTEQDSKD STYSLSSTLT LSKADYEKHK VYACEVTHQG
LSSPVTKSFN RGEC,

SEQ ID NO: 13 for the amino acid sequence of RAM4hc:

EVQLLESGGG LVQPGGSLRL SCAASGFTFS IYAMDWVRQA PGKGLEWVSG IVPSGGFTKY
ADSVKGRFTI SRDNSKNTLY LQMNSLRAED TAVYYCARVN VIAVAGTGYY YYGMDVWGQG
TTVTVSSAST KGPSVFPLAP SSKSTSGGTA ALGCLVKDYF PEPVTVSWNS GALTSGVHTF PAVLQSSGLY
SLSSVVTVPS SSLGTQTYIC NVNHKPSNTK VDKRVEPKSC DKTHTCPPCP APELLGGPSV FLFPPKPKDT
LMISRTPEVT CVVVDVSHED PEVKFNWYVD GVEVHNAKTK
PREEQYNSTY RVVSVLTVLH QDWLNGKEYK CKVSNKALPA PIEKTISKAK GQPREPQVYT LPPSREEMTK
NQVSLTCLVK GFYPSDIAVE WESNGQPENN YKTTPPVLDS DGSFFLYSKL TVDKSRWQQG
NVFSCSVMHE ALHNHYTQKS LSLSPGK,

SEQ ID NO: 14 for the amino acid sequence of RAM4lc:

DIQMTQSPGT LSLSPGERAT LSCRASQGVS SSSLAWYQQK PGQAPRLLIY GTSSRATGIP DRFSGSASGT
DFTLTISRLQ PEDFAVYYCQ QYGRSLTFGG GTKVEIKRTV AAPSVFIFPP SDEQLKSGTA SVVCLLNNFY
PREAKVQWKV DNALQSGNSQ ESVTEQDSKD STYSLSSTLT LSKADYEKHK VYACEVTHQG
LSSPVTKSFN RGEC.

The anti-MIF antibody of the invention is preferably an isolated monoclonal antibody. The anti-MIF antibody can be an IgG, an IgM, an IgE, an IgA, or an IgD molecule. In other embodiments, the anti-MIF antibody is an IgG1, IgG2, IgG3 or IgG4 subclass. In other embodiments, the antibody is either subclass IgG1 or IgG4. In other embodiments, the antibody is subclass IgG4. In some embodiments, the IgG4 antibody has a single mutation changing the serine (serine228, according to the Kabat numbering scheme) to proline. Accordingly, the CPSC sub-sequence in the Fc region of IgG4 becomes CPPC, which is a sub-sequence in IgG1 (Angal et al. Mol Immunol. 1993, 30, 105-108).

Additionally, the production of anti-(ox)MIF antibodies may include any method known in the art for the purification of an antibody, e.g., via anion exchange chromatography or affinity chromatography. In one embodiment the anti-(ox)MIF antibody can be purified from cell culture supernatants by size exclusion chromatography.

The terms "center region" and "C-terminal region" of MIF refer to the region of human MIF comprising amino acids 35-68 and aa 86-115, respectively, preferably aa 50-68 and aa 86 to 102 of human MIF, respectively. Particularly preferred antibodies of the present invention bind to either region aa 50-68 or region aa 86-102 of human MIF. This is also reflected by the preferred antibodies of the invention, like RAB0, RAB4 RAB2 and RAB9 as well as RAM4, RAM9 and RAM0 which bind as follows:

RAB4 and RAM4:    aa 86-102
RAB9 and RAM9:    aa 50-68
RAB0 and RAM0:    aa 86-102
RAB2: aa 86-102

The term "epitope" includes any protein determinant capable of specific binding to an immunoglobulin or an antibody fragment. Epitopic determinants usually consist of chemically active surface groupings of molecules such as exposed amino acids, amino sugars, or other carbohydrate side chains and usually have specific three-dimensional structural characteristics, as well as specific charge characteristics.

The term "vector" refers to a nucleic acid molecule capable of transporting another nucleic acid to which it has been linked. In some embodiments, the vector is a plasmid, i.e., a circular double stranded DNA loop into which additional DNA segments may be ligated.

The term "host cell" refers to a cell line, which is able to produce a recombinant protein after introducing an expression vector. The term "recombinant cell line", refers to a cell line into which a recombinant expression vector has been introduced. It should be understood that "recombinant cell line" means not only the particular subject cell line but also the progeny of such a cell line. Because certain modifications may occur in succeeding generations due to either mutation or environmental influences, such progeny may not, in fact, be identical to the parent cell, but are still included within the scope of the term "recombinant cell line" as used herein.

The host cell type according to the present invention is e.g. a COS cell, CHO cell or, e.g., an HEK293 cell, or any other host cell known to a person skilled in the art, thus also for example including bacterial cells, like e.g. *E.coli* cells. In one embodiment, the anti-MIF antibody is expressed in a DHFR-deficient CHO cell line, e.g., DXB11, and with the addition of G418 as a selection marker. When recombinant expression vectors encoding antibody genes are introduced into CHO host cells, the antibodies are produced by culturing the host cells for a period of time sufficient to allow for expression of the antibody in the host cells or secretion of the antibody into the culture medium in which the host cells are grown.

Anti-(ox)MIF antibodies can be recovered from the culture medium using standard protein purification methods.

The second active ingredient of the combination therapy as provided by the present invention is a chemotherapeutic.

Chemotherapeutic agents in the general sense thereof, are compounds, which can be used for the treatment of a disease or disorder that arises from bacterial, viral or parasitic infection or that is due to transformation of normal cells (cancer). One particular indication of chemotherapy is cancer. Chemotherapeutic agents can act for example by killing cells that divide more rapidly than other cells, and thus target cancer cells which commonly divide more rapidly than non-cancerous cells. Most chemotherapeutic agents work by impairing cell division at one of several stages of the cell cycle. Thus, they are able to target those cells that divide more rapidly. Chemotherapeutic agents can be either cytostatic, i.e., they slow down or abrogate the growth or division of cells; other chemotherapeutic agents can cause damage to cells and kill them; in that case they are termed cytotoxic. Most cytotoxic drugs inflict a damage that *per se* does not suffice to kill a cell but that generates a stimulus to initiate programmed cell death (apoptosis).

In general, major classes of chemotherapeutic drugs are alkylating agents, anti-metabolites, anthracyclines, plant alkaloids, topoisomerase inhibitors and other anti-tumour agents. Most commonly, as mentioned above, these drugs affect one or several stages of the cell cycle; they can also affect DNA synthesis or DNA integrity. Other chemotherapeutics do not directly interfere with DNA. These are newer classes of chemotherapeutics and can include monoclonal antibodies and tyrosine kinase inhibitors like imatinib mesylate. Other examples are chemotherapeutic hormones and hormone antagonists, e.g. glucocorticosteroids.

Examples for alkylating agents, which alkylate nucleophilic functional groups are mechlorethamine, cyclophosphamide, chlorambucil, melphalane, trofosfamide, ifosfamide, carmustine, lomustine, dacarbazine, temozolomide, mitomycine C and many others. Cisplatin, carboplatin, oxaliplatin and other platinum containing compounds form stable complexes with DNA.

Cytotoxic anti-metabolites are folic acid analogues (e.g., methotrexat/aminopterin, raltitrexed, pemetrexed), purine analogs (e.g., 6-mercaptopurine, azathioprine, thioguanine, fludarabine, cladribine) or pyrimidine analogs (cytarabine, gemcitabine, 5-fuloruracil and its prodrugs, deazacytidine). Antimetabolites either inhibit DNA-synthesis by interfering with crucial steps in the *de novo* synthesis of purine and pyrimidine nucleotides or they become incorporated into DNA during the S-phase of the cell cycle, where they interfere with DNA-folding, DNA-repair or methylation. Alternatively, some compounds also become incorporated into RNA. Examples for alkaloids and terpenoids which are derived from plants and block cell division by preventing microtubule function are vinca-alkaloids and taxanes. Particularly well known vinca-alkaloids are vincristine, vinblastine, vinorelbine and vindesine. Podophyllotoxin is an additional example of a plant-derived compound. An example for a taxane is docetaxel or paclitaxel. Another example is abraxane, an albumin bound paclitaxel. Estramustin is an example of a synthetic compound that targets tubulin.

Examples of topoisomerase inhibitors, which are inhibitors of enzymes that maintain the topology of DNA, include camphtotecines like irinotecan and topotecan (type 1 topoisomerase inhibitors) or amsacrin, etoposide, etoposide phosphate and teniposide (topoisomerase-type 2 inhibitors). Finally, examples of antineoplastic intercalating agents include dactinomycin, doxorubicin, epirubicin, bleomycin and others.

A comprehensive overview is comprised in Goodman and Gilman, The Pharmacological Basis of Therapeutics, 12th Edition, "General Principles of Cancer Chemotherapy".

The following are examples for alkylating agents:

Mechlorethamine
Cyclophosphamide Ifosfamide
Melphalan
Chlorambucil
Procarbazine (N-methylhydrazine, MIH)
Busulfan
Camustine (BCNU)
Streptozocin
(streptozotocin)
Bendamustine
Dacarbazine (DTIC; dimethyltriazenol midazole carboxamide)
Temozolomide
Cisplatin, carboplatin, oxaliplatin
Antimetabolites are exemplary represented by
Methotrexate (Amethopterin)
Pemetrexed
Fluorouracil (5-fluorouracil; 5-FU), capecitabine
Cytarabine (cytosine arabinoside)
Gemcitabine
5-aza-cytidine
Deoxy-5-aza-cytidine
Mercaptopurine (6-mercaptopurine; 6-MP)
Pentostatin (2'-deoxycoformycin)
Fludarabine
Clofarabine
Nelarabine

while Natural Products can be selected from:

Vinblastine
Vinorelbine
Vincristine
Paclitaxel, docetaxel
Etoposide
Teniposide

Topotecan

Irinotecan

Dactinomycin

(actinomycin D)

Daunorubicin

(daunomycin, rubidomycin)

Doxorubicin

Yondelis

Mitoxantrone

Bleomycin

Mitomycin C

L-Asparaginase

Examples for Hormones and Antagonists are:

Mitotane (o.*p'*DDD)

Prednisone

Hydroxyprogesterone caproate,

medroxyprogesterone acetate, megestrol acetate

Dietyhlstilbestrol, ethinyl estradiol

Tamoxifen, toremifene

Anastrozole, letrozole, exemestane

Testosterone propionate, fluoxymesterone

Flutamide, casodex

Leuprolide

while examples for further agents are: Hydroxyurea

Tretinoin, arsenic trioxide

Histone deacetylase inhibitor (vorinostat) Imatinib

Dasatinib, nilotinib

Gefitinib, erlotinib

Sorafenib

Sunitinib

Lapatinib

Bortezomib

Interferon-alfa,

Interleukin-2

Thalidomide

Lenalidomide

Temsirolimus,

Everolimus

Chemotherapeutics have been shown to be successful in alleviation and treatment of cancer. However, most chemotherapeutics are associated with a range of side effects which are in some cases extreme, to the extent that the treatment has to be abrogated. In any case, the side effects place a further burden on the physical and mental health of a patient and should thus be avoided as far as possible.

With the present invention, by combining a chemotherapeutic with an anti-MIF antibody, it is now possible to reduce the amount of the chemotherapeutic agent which is necessary for a given treatment compared to a situation where the chemotherapeutic agent is given as the sole active ingredient. A further possibility enabled by the present invention is to maintain the dose of the chemotherapeutic as compared to the chemotherapeutic given alone and have a much higher treatment response in the patient.

This increase of the treatment response in the patient also indicates the possibility to achieve a treatment response as with a chemotherapeutic alone, with a combination of anti-MIF antibody with a lower dose of chemotherapeutic agent, e.g. in cases where the side effects of the chemotherapeutic do not allow continuous treatment with the higher dose.

It was shown quite surprisingly by the present inventors that the effect obtained by combining a chemotherapeutic with an anti-MIF antibody showed a much higher treatment response than with either the anti-MIF antibody or the chemotherapeutic agent alone.

A treatment response can easily be determined by a person skilled in the art and refers to diminishing or ameliorating or alleviating a given condition. Methods to determine such a treatment response are well known and can be for example determination of the likelihood or length of survival of a subject having a disease and being treated with a combination of MIF antagonist and chemotherapeutic agent with the likelihood or length of survival in other subjects having the same disease and being treated with either agent alone, or by determining the change of symptoms within one and the same patient over a period of time. An example well known to a person skilled in the art is the Kaplan-Meier-Plot.

Other methods/assays are well known and can be derived for example from general textbooks, like The Pharmacological Basis of Therapeutics, 12th Edition, "General Principles of Cancer Chemotherapy. Introduction". This reference is incorporated hereby in its entirety by reference; additional methods/assays are those as described in the present examples.

Preferred chemotherapeutics according to the present invention are doxorubicin and gemcitabine. Doxorubicin can be used in a preferred embodiment in combination with cisplatin.

Also preferred is e.g. abraxane.

Particularly preferred combinations are

- a treatment of ovarian cancer with doxorubicin, preferably in combination with cisplatin, together with an anti-MIF antibody, or
- a treatment of pancreatic carcinoma with gemcitabine and/or abraxane, preferably together with an anti-MIF antibody.

In a preferred embodiment of the above combinations the anti-MIF antibody is selected from the group of RAB9, RAB4 and RAB0.

"Cancer" in the present context encompasses all disorders or diseases in which a cell or a group of cells displays uncontrolled growth, invasion (intrusion and destruction of adjacent tissues) and sometimes metastasis.

Further, in a preferred embodiment, the cancer can be MIF-related. MIF-related cancers are e.g. lymphoma, sarcoma, prostatic cancer and colon cancer, bladder cancer, pancreas cancer, ovarian cancer, melanoma, hepatocellular carcinoma, ovarian cancer, breast cancer and pancreatic cancer, as well as endometriosis. Possible dosage forms which are envisaged by the present application are tablets, capsules, sachets or pills. The granules can be used as such as a preferred dosage form, can be filled into capsules or sachets or can be further compressed into tablets or pills.

Further dosage forms which are also encompassed by the present application are drinks or syrups, elixirs, tinctures, suspensions, solutions, hydrogels, films, lozenges, chewing gums, orally disintegrating tablets, mouth-washes, toothpaste, lip balms, medicated shampoos, nanosphere suspensions and microsphere tablets, as well as aerosols, inhalers, nebulisers, smoking or freebase powder forms and dosage forms for topical application like creams, gels, liniments or balms, lotions, ointments, ear drops, eye drops and skin patches.

Further encompassed are suppositories which can be used e.g. rectally or vaginally. All these dosage forms are well-known to a person skilled in the art.

Preferred dosage forms according to the present invention are oral forms like granules, coated granules, tablets, enteric coated tablets, pellets, suppositories and emulsions. Even more preferred are granules and tablets. Other preferred dosage forms are parenteral or topical dosage forms. A particular preferred administration route for the anti MIF antibody is a subcutaneous or intravenous application. A preferred administration route for the chemotherapeutic agent is oral application (e.g., a granule, liquid, sachet or tablet). A further preferred application form for the chemotherapeutic is topical application, wherein a topical application can encompass an application to the skin and/or a spray, like a nasal spray or inhaler. A further preferred administration route for a chemotherapeutic is an intravenous application or an application via a subcutaneous injection (including slow release formulations).

The administration can however principally be by all known routes.

The term "combination" or "combination therapy" are used interchangeably here. They refer to a dosing regimen where the anti-MIF antibody is administered together with or sequentially to the chemotherapeutic or *vice versa*. The dosing regimen would be typically daily for chemotherapeutics and every 2 weeks for the anti-MIF antibody. Preferred dosing regimens are:

As explained above, it is possible to administer the anti-MIF antibody together with the chemotherapeutic agent(s) or sequentially. "Together with" in this context means that not more than 10 minutes have passed between the administration of the anti-MIF antibody and the administration of the chemotherapeutic.

"Sequentially" means that more than 10 minutes have passed between the administration of the anti-MIF antibody and the administration of the chemotherapeutic agent. The time period can then be more than 10 min., more than 30 minutes, more than 1 hour, more than 3 hours, more than 6 hours or more than 12 hours.

Anti-MIF antibody and chemotherapeutic agents are principally dosed in a way to ensure that both compounds are present within the body during the same time period (for a certain time span). An anti-MIF antibody has a half-life of typically 2 - 4 weeks, chemotherapeutic agents a half-life of 2-48 hours.

Therefore, the above combination therapy also explicitly encompasses a sequential dosing regime where the skilled person takes into account the well known half life of the respective chemotherapeutic drug in question and the antibody in question. In view of the fact that antibodies generally have a half-life of 2 - 4 weeks, administration of the antibody in question could be only every 2 weeks, every 3 weeks or once a month. The chemotherapeutic drug to be administered in the inventive combination therapy with such an antibody has in a typical embodiment a half-life of 2 - 48 h; therefore, administration of the chemotherapeutic could be every 5 hours, every 6 hours, three times a day, twice a day, once daily, once a week or once per three week cycle in a typical embodiment.

Dosing of chemotherapeutics agent, as well as the combined dosing with antibodies, according to the present invention, however, will need to be determined by the practitioner on a case-by-case basis according to the specific disorder to be treated and the particulars of the afflicted subject. The person of skill in the art is aware of the respective guidelines for a given chemotherapeutic agent.

As a general understanding in curative chemotherapy, one would wish to apply the highest tolerated dose to achieve the desired dose intensity. The dose is reduced only if there is toxicity (i.e., neutrophil counts < 4000 (but >2500) = administer half the dose, see cisplatin or cyclphosphamide). Most chemotherapeutic agents are administered on the basis of (m)g/m$^2$ body surface. Differences in tolerance and efficacy between mouse, rat and man are typically accounted for by basing the dose on body surface.

In a particularly preferred embodiment, the active ingredient would be an ingredient which should be delivered with a controlled, e.g. a delayed release. That is, the orally administrable dosage forms of the present invention comprising such an active ingredient might be provided with a coating. Thus, in a preferred embodiment the present invention is directed to granules with coatings and in particular to granules comprising active ingredients which shall be released in a controlled manner, whereby these granules have a coating.

More preferred, this coating is pharmacologically acceptable coating and particularly preferred is an enteric coating, a prolonged release coating or a delayed release coating; all such coatings are well known to a person skilled in the art.

A subset of *in vivo* protective anti-MIF mAbs (e.g. RAB9, RAB4, RAB0), which are directed against the pro-inflammatory cytokine MIF (Macrophage Migration Inhibitory Factor) do not bind to unmodified MIF in its reduced state. By contrast, these mAbs were shown to be highly selective for a redox dependent MIF isoform. A particularly preferred antibody is antibody RAB9.

Another particularly preferred antibody is antibody RAB4.

Yet another particularly preferred antibody is antibody RAB0.

A very preferred antibody is antibody RAM9.

As is shown by the present invention, the combination therapy proposed here is advantageous in that it results in a synergistic effect of both components.

The present invention will be in the following described by way of the examples, whereby the examples shall be considered by no means as limiting the present invention.

### REFERENCE EXAMPLES

A) GCO-assay for antibody screening:

A THP1 suspension culture is centrifuged and cells are resuspended in fresh full medium to a cell density of 10$^6$ cells per ml. This culture is transferred into wells of a 96-well microplate (90 μl/well) and a potential anti-MIF antibody is added to give a final concentration of 75 μg/mL Each antibody is tested in triplicate. After o/n incubation at 37°C dexamethasone is added to give a concentration of 2 nM and after one hour incubation at 37°C LPS is added (3 ng/ml final concentration). After further six hours incubation at 37°C the supernatant is harvested and the IL-6 concentrations are determined in a commercially available ELISA. The results of the triplicates are averaged and the percentage of IL-6 secretion is determined in comparison to the control antibodies. Antibodies that result in an IL-6 secretion of less than 75% are evaluated as positive.

B) Assay for determination of IC$_{50}$ values

The experimental procedure is carried out as described for the screening assay with the exception that increasing amounts of antibody are used (typically from 1 - 125 nM). The resultant dose response curve is expressed as % inhibition in comparison to a negative control antibody. This curve is used for calculation of the maximum inhibitory effect of the antibody (%Inh max) and the antibody concentration that shows 50% of the maximum inhibitory effect (IC$_{50}$).

C) Inhibition of cell proliferation

Serum stimulates secretion of MIF in quiescent NIH/3T3 and MIF in turn stimulates cell proliferation. Antibodies inhibiting this endogenous MIF, therefore, decrease the proliferation of quiescent NIH/3T3 cells. The reduction of proliferation is determined by the incorporation of $^3$H-thymidine.

1000 NIH/3T3 cells per well are incubated in a 96 well plate over the weekend at 37°C in medium containing 10% serum. Cells are then starved over night at 37°C by incubation in medium containing 0.5% serum. The 0.5% medium is removed and replaced by fresh medium containing 10% serum, 75 $\mu$g/ml antibody and 5$\mu$ Ci/ml of 3H-thymidine. After 16 hours incubation in a $CO_2$ incubator at 37°C cells are washed twice with 150 $\mu$l of cold PBS per well. Using a multi-channel pipette 150 $\mu$l of a 5% (w/v) TCA solution per well are added and incubated for 30 minutes at 4°C. Plates are washed with 150 $\mu$l PBS. Per well 75 $\mu$l of a 0.5M NaOH solution with 0.5% SDS are added, mixed and stored at room temperature. Samples are measured in a ß-counter by mixing 5 ml of Ultima Gold (Packard) and 75 $\mu$l sample solution. Each determination is done in triplicate and the values are compared with the values of the control antibody by a t-test. Antibodies that significantly reduce proliferation ($P<0.05$) are evaluated as positive.

D) Binding studies: Epitope determination of anti-MIF antibodies

Each peptide is diluted in coupling buffer to give a peptide concentration of typically 1 $\mu$g/ml added to microplates (NUNC Immobilizer™ Amino Plate F96 Clear) and incubated over night at 4°C (100 $\mu$l/well). As controls recombinant ful1 length MIF and PBS are used. The plate is washed 3 times with 200 $\mu$l PBST and antibodies (2-4 $\mu$g/ml in PBS) are added (100 $\mu$l/well) and incubated for 2 hours at room temperature with gentle shaking. The plate is washed 3 times with 200 $\mu$l PBST and detection antibody (e.g. Fc specific antihuman IgG/HRP labelled, Sigma) is added (100 $\mu$l/well). After incubation for 1 hour at room temperature with gentle shaking, the plate is washed 3 times with 200 $\mu$l PBST. Each well is incubated with 100 $\mu$l TMB (3,3',5,5'-tetramethylbenzidine) solution (T-0440, Sigma) for 30 minutes in the dark. Staining reaction is stopped by adding 100 $\mu$l of 1.8 M $H_2SO_4$-solution per well. Samples are measured at 450 nm.

E) Affinity determination of Fab fragments of anti-MIF antibodies by Biacore

Typically, 40 RU units of human recombinant MIF are immobilized on a sensor chip with a CM5 (= carboxymethylated dextran) matrix (Biacore). Fab fragments are injected at a concentration range of typically 6 - 100 nM diluted in HBS-EP. After each cycle the chip is regenerated with 50 mM NaOH + 1M NaCl. Affinities are calculated according to the 1:1 Langmuir model.

**EXAMPLES**

Introduction:

Therapy of cancer and hyperproliferative disorders using chemotherapeutic drugs is often hampered by severe side effects or a required increase of dosage. In addition, many tumors have proven to be resistant to chemotherapeutic intervention. In this invention we describe that the effectiveness of chemotherapeutic compounds (e.g., gemcitabine, doxorubicin, or cisplatin) can be increased by combination with a MIF antagonist (e.g. an anti-oxMIF antibody). Such a combination has the potential to result in an improved therapy of cancer and hyperproliferative disorders in comparison to the respective monotherapy and to prolong significantly the life expectancy of patients.

**OVERVIEW**

| Tumor | Chemotherapy | Antibody | *In vivo/in vitro* | Effect |
|---|---|---|---|---|
| | | | | |
| | | | | |
| Ovarian cancer | Doxorubicin | RAM0 | *In vitro* | Synery |
| | | RAM9 | *In vitro* | Synergy |
| | Cisplatin | RAM0 | *In vitro* | Synergy |
| | | RAM0 | *In vivo* | Synergy |
| Prostate cancer | Mitoxantrone | RAM0 | *In vitro* | Synergy |

**ANTI-MIF IN COMBINATION WITH INTERCALATING AGENTS**

(Exemplified by a combinations of an anti-MIF antibody with **doxorubicin)**

**Example 1: Induction of apoptosis in doxorubicin-resistant human ovarian cancer cells by combined application of doxorubicin and anti-MIF antibodies:**

In an *in vitro* assay, doxorubicin resistant A2780adr ovarian cancer cells were treated either with anti-MIF antibodies RAM9 or RAM0 alone or in combination with doxorubicin. The induction of apoptosis was assessed by detection of caspase 3 activity after 72 hours of treatment. Non-treated cells or cells treated with antibody alone did not show any enhanced caspase 3 activity compared to isotype control antibody treated or untreated cells. Doxorubicin induced a significant but minor enhanced caspase 3 activity. The combination of anti-MIF antibodies and doxorubicin further enhanced caspase 3 activity and therefore the induction of apoptosis. The results show, that a previously doxorubicin resistant cell line becomes sensitive to this chemotherapy drug by combined application with MIF neutralizing antibodies. Combination of Doxorubicin with RAM0 (Figure 1A) or RAM9 (Figure 1 B) gave comparable results. These results confirm the synergistically increased caspase activity observed in vivo when anti-MIF is combined with an antimetabolite.

**ANTI-MIF IN COMBINATION WITH ALKYLATING AGENTS**

(Exemplified by a combinations of an anti-MIF antibody with **cisplatin)**

**Example 2: Anti-MIF antibody sensitizes A2780 ovarian cancer cells to the action of *cisplatin in vitro:***

The synergistic effect of cisplatin and an anti-MIF antibody was demonstrated in a cisplatin-dependent cell killing assay. A2780 cells were incubated with increasing concentrations of cisplatin either in the absence or in presence of 50 nM RAM0 or a human isotype control antibody. After 48 hours of incubation cells were detached with Accutase™, labeled with calcein-AM and the level of calcein fluorescence was determined by flow cytometry. The mean fluorescence intensity in the absence of any drug or antibody was set to 1 to normalize for interassay variations. The mean fluorescent intensities were blotted against the cisplatin concentration and the half maximum active concentrations of cisplatin (EC50-values) were calculated. The EC50-value was reduced significantly (p<0.01) when cisplatin was combined with RAM0 (Figure 2). The anti-MIF antibody as a monotherapy (in absence of cisplatin) has no effect on cell killing in comparison to the isotype control antibody (data not shown).

**Example 3: Anti-MIF antibody sensitizes A2780 ovarian cancer cells to the action of cisplatin *in vivo:***

The data summarized above showed that, in cell culture, RAM0 rendered ovarian cancer cells more susceptible to cell killing by cisplatin. This effect is of high relevance, in particular if it can also be observed *in vivo.* This was explored in female Mf1 nude mice inoculated with A2780 xenografts (Figure 3). Mice (n= 10/group) were inoculated with $1*10^6$ A2780 cells resuspended in matrigel. RAM0 (15 mg/kg) or a human isotype control antibody (15 mg/kg) was injected every other day starting one day after inoculation of the xenograft. Cisplatin was administered once a week starting on day 2 after xenograft inoculation. Cisplatin did not have any effect on tumor growth as a monotherapy. RAM0 had a minor but not significant effect. However, RAM0 sensitizes the tumor to cisplatin treatment and the combination of cisplatin and RAM0 shows a significantly reduced tumor growth (p=0.04).

**ANTI-MIF IN COMBINATION WITH INTERCALATING AGENTS/NATURAL AGENTS**

(Exemplified by a combinations of an anti-MIF antibody with mitoxantrone)

**Example 4: LnCAP prostate cancer cells are sensitized to the cytotoxic action of mitoxantrone by anti-MIF antibody RAM0**

The synergistic effect of mitoxantrone and an anti-MIF antibody was demonstrated in a mitoxantrone-dependent cell killing assay. LnCAP cells were incubated with increasing concentrations of mitoxantrone either in the absence or in presence of 100 nM RAM0 or a human isotype control antibody. After 48 hours of incubation cells were detached with Accutase™, labeled with calcein-AM and the level of calcein fluorescence was determined by flow cytometry. The mean fluorescence intensity in the absence of any drug or antibody was set to 1 to normalize for interassay variations. The mean fluorescent intensities were plotted against the mitoxantrone concentration and the half maximum active concen-

trations of mitoxantrone ($EC_{50}$-values) were calculated. 10 independent $EC_{50}$ values were determined for each mitoxantrone/antibody combination (n=10) and the results are depicted in Figure 4. The mean $EC_{50}$-value for mitoxantrone alone was determined to be 1.6 nM. The combination of mitoxantrone and a control antibody gave the identical mean $EC_{50}$. However, the mean $EC_{50}$ was significantly reduced to 0.97 nM when mitoxantrone was combined with RAM0. The anti-MIF antibody as a monotherapy (in absence of mitoxantrone) has no effect on cell killing in comparison to the isotype control antibody (data not shown).

Conclusions

The strong enhanced efficacy of combining a MIF antibody with standard of care chemotherapeutic drugs has been shown in the above different experiments. In an *in vitro* assay, a doxorubicin-resistant cell line was rendered doxorubicin-sensitive by co-incubation with anti-MIF antibodies as demonstrated by the increase of caspase activity. In addition, anti-MIF antibody cisplatin resistant cancer cells were rendered cisplatin sensitive in an *in vivo* model. Furthermore, in an *in vitro* assay, a significant reduction of mean $EC_{50}$ could be shown for a combination of mitoxantrone and an anti-MIF antibody. This is the first description of a synergistic effect of chemotherapeutic drugs with MIF-inhibitors. A combination therapy using chemotherapeutic agents and MIF antibodies can thus overcome current limitations of chemotherapeutic intervention and improve the therapeutic result in patients with hyperproliferative disorders.

**Claims**

1. An anti-MIF antibody in combination with a chemotherapeutic agent for use in the treatment of cancer.

2. The anti-MIF antibody in combination with chemotherapeutic agent according to claim 1 for use in the treatment of cancer, wherein the cancer is selected from the following group: pancreatic cancer, ovarian cancer, prostate cancer, breast cancer, lung cancer and colon cancer, more preferred pancreatic cancer, prostate cancer and ovarian cancer.

3. The antibody in combination with a chemotherapeutic agent according to claim 1 or 2 wherein the anti-MIF antibody is selected from the following group: anti-MIF antibody RAM9, RAM0 and/or RAM4.

4. The antibody in combination with a chemotherapeutic agent according to claim 1, 2 or 3, wherein the chemotherapeutic is selected from mitoxantrone, doxorucibin, and/or cisplatin.

5. The antibody in combination with a chemotherapeutic agent according to any of claims 1 to 4, the anti-MIF antibody is RAM9, the chemotherapeutic is doxorubicin, optionally in combination with cisplatin and the cancer is ovarian cancer.

6. The antibody in combination with a chemotherapeutic agent according to any of claims 1 to 4, the anti-MIF antibody is RAM0, the chemotherapeutic is doxorubicin, optionally in combination with cisplatin and the cancer is ovarian cancer.

7. The antibody in combination with a chemotherapeutic agent according to any of claims 1 to 4, the anti-MIF antibody is RAM4, the chemotherapeutic is doxorubicin, optionally in combination with cisplatin and the cancer is ovarian cancer.

8. The antibody in combination with a chemotherapeutic agent according to any of claims 1 to 4, the anti-MIF antibody is RAM0, the chemotherapeutic is mitoxantrone, and the cancer is prostate cancer.

9. A kit comprising the antibody in combination with a chemotherapeutic agent as defined in any of claims 1 to 8 above, and instructions for use.

10. An antibody in combination with a chemotherapeutic agent as defined in any of claims 1 to 8 above, or the kit of claim 9, for use in the treatment of cancer.

Figure 1

A

**% Increase of caspase 3 activity**

B

**% Increase of caspase 3 activity**

Figure 2

Figure 3

**Combination with Mitoxantrone**

Figure 4

Europäisches
Patentamt

European
Patent Office

Office européen
des brevets

# EUROPEAN SEARCH REPORT

Application Number

EP 16 16 5526

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 01/64749 A2 (IDEC PHARMA CORP [US]) 7 September 2001 (2001-09-07) * the whole document * * in particular * * abstract * * page 1, line 10 - page 6, line 25 * * page 34, line 10 - page 40, line 5 * * claims 1-53; figures 1-16; examples 1-9 * | 1-10 | INV. A61K39/395 C07K16/24 A61K31/7068 A61K31/704 A61K33/24 A61K31/137 A61P35/00 A61K31/136 A61K39/00 |
| X,D | WO 2009/086920 A1 (BAXTER INT [US]; BAXTER HEALTHCARE SA [CH]; DYAX CORP [US]; KERSCHBAUM) 16 July 2009 (2009-07-16) * the whole document * * in particular * * abstract * * page 3, line 11 - line 10 * * page 13, line 9 - page 16, line 25 * * claims 1-22; figures 1-10; examples 1-16 * | 1-10 | |
| X | WO 94/26307 A1 (PICOWER INST MED RES [US]) 24 November 1994 (1994-11-24) * the whole document * | 1-10 | TECHNICAL FIELDS SEARCHED (IPC) A61K C07K A61P |
| X | CN 1 869 207 A (INST OF BIOPHYSICS CAS [CN] INST BIOPHYSICS CN ACAD SCI [CN]) 29 November 2006 (2006-11-29) * the whole document * | 1-10 | |
| A | US 2003/099653 A1 (BUCALA RICHARD J [US] ET AL) 29 May 2003 (2003-05-29) * the whole document * | 1-10 | |

-/--

The present search report has been drawn up for all claims

| Place of search | Date of completion of the search | Examiner |
|---|---|---|
| Munich | 11 July 2016 | Bretherick, James |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

Europäisches
Patentamt

European
Patent Office

Office européen
des brevets

## EUROPEAN SEARCH REPORT

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | ISHIGURO Y ET AL: "Macrophage migration inhibitory factor has a proinflammatory activity via the p38 pathway in glucocorticoid-resistant ulcerative colitis", CLINICAL IMMUNOLOGY, ACADEMIC PRESS, US, vol. 120, no. 3, 1 September 2006 (2006-09-01), pages 335-341, XP024941335, ISSN: 1521-6616, DOI: 10.1016/J.CLIM.2006.05.010 [retrieved on 2006-09-01] * the whole document * | 1-10 | |
| A | WO 2007/075270 A2 (IBC PHARMACEUTICALS INC [US]; CHANG CHIEN-HSING [US]; GOLDENBERG DAVID) 5 July 2007 (2007-07-05) * the whole document * | 1-10 | |
| A | SHIMIZU T ET AL: "HIGH EXPRESSION OF MACROPHAGE MIGRATION INHIBITORY FACTOR IN HUMAN MELANOMA CELLS AND ITS ROLE IN TUMOR CELL GROWTH AND ANGIOGENESIS", BIOCHEMICAL AND BIOPHYSICAL RESEARCH COMMUNICATIONS, ACADEMIC PRESS INC. ORLANDO, FL, US, vol. 264, no. 3, 2 November 1999 (1999-11-02), pages 751-758, XP001008703, ISSN: 0006-291X, DOI: 10.1006/BBRC.1999.1584 * the whole document * | 1-10 | TECHNICAL FIELDS SEARCHED (IPC) |
| A | WO 98/17314 A1 (PICOWER INST MED RES [US]) 30 April 1998 (1998-04-30) * the whole document * | 1-10 | |

-/--

The present search report has been drawn up for all claims

| Place of search | Date of completion of the search | Examiner |
|---|---|---|
| Munich | 11 July 2016 | Bretherick, James |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

................................................................

& : member of the same patent family, corresponding
document

EPO FORM 1503 03.82 (P04C01)

Europäisches
Patentamt

European
Patent Office

Office européen
des brevets

# EUROPEAN SEARCH REPORT

Application Number

EP 16 16 5526

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 2011/090492 A1 (IMMUNOMEDICS INC [US]; CHANG CHIEN-HSING [US]; LOSMAN MICHELE J [US];) 28 July 2011 (2011-07-28) * the whole document * ----- | 1-10 | |
| A | US 2011/158905 A1 (GOLDENBERG DAVID M [US] ET AL) 30 June 2011 (2011-06-30) * the whole document * ----- | 1-10 | |

TECHNICAL FIELDS
SEARCHED (IPC)

The present search report has been drawn up for all claims

| Place of search | Date of completion of the search | Examiner |
|---|---|---|
| Munich | 11 July 2016 | Bretherick, James |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 16 16 5526

This annex lists the patent family members relating to the patent documents cited in the above-mentioned European search report.
The members are as contained in the European Patent Office EDP file on
The European Patent Office is in no way liable for these particulars which are merely given for the purpose of information.

11-07-2016

| Patent document cited in search report | | | Publication date | Patent family member(s) | | | Publication date |
|---|---|---|---|---|---|---|---|
| WO 0164749 | A2 | | 07-09-2001 | AU | 3867701 | A | 12-09-2001 |
| | | | | US | 2003235584 | A1 | 25-12-2003 |
| | | | | US | 2006191024 | A1 | 24-08-2006 |
| | | | | WO | 0164749 | A2 | 07-09-2001 |
| WO 2009086920 | A1 | | 16-07-2009 | AU | 2008346517 | A1 | 16-07-2009 |
| | | | | BR | PI0821682 | A2 | 16-06-2015 |
| | | | | CA | 2711029 | A1 | 16-07-2009 |
| | | | | CN | 101983207 | A | 02-03-2011 |
| | | | | CN | 103724430 | A | 16-04-2014 |
| | | | | DK | 2231707 | T3 | 02-03-2015 |
| | | | | EP | 2231707 | A1 | 29-09-2010 |
| | | | | EP | 2548890 | A1 | 23-01-2013 |
| | | | | EP | 2754671 | A2 | 16-07-2014 |
| | | | | ES | 2531629 | T3 | 18-03-2015 |
| | | | | HK | 1147762 | A1 | 07-08-2015 |
| | | | | HR | P20150224 | T1 | 05-06-2015 |
| | | | | IL | 206715 | A | 30-06-2015 |
| | | | | JP | 5502752 | B2 | 28-05-2014 |
| | | | | JP | 2011510616 | A | 07-04-2011 |
| | | | | KR | 20100102197 | A | 20-09-2010 |
| | | | | NZ | 586600 | A | 25-05-2012 |
| | | | | NZ | 596409 | A | 31-05-2013 |
| | | | | NZ | 611117 | A | 31-10-2014 |
| | | | | PT | 2231707 | E | 25-03-2015 |
| | | | | RU | 2010132647 | A | 10-02-2012 |
| | | | | RU | 2013153592 | A | 10-06-2015 |
| | | | | SI | 2231707 | T1 | 30-04-2015 |
| | | | | US | 2009220521 | A1 | 03-09-2009 |
| | | | | US | 2010260768 | A1 | 14-10-2010 |
| | | | | US | 2015023978 | A1 | 22-01-2015 |
| | | | | WO | 2009086920 | A1 | 16-07-2009 |
| WO 9426307 | A1 | | 24-11-1994 | AT | 474597 | T | 15-08-2010 |
| | | | | AU | 692159 | B2 | 04-06-1998 |
| | | | | AU | 6834594 | A | 12-12-1994 |
| | | | | CA | 2163211 | A1 | 24-11-1994 |
| | | | | EP | 0702566 | A1 | 27-03-1996 |
| | | | | EP | 1741779 | A2 | 10-01-2007 |
| | | | | JP | 4708510 | B2 | 22-06-2011 |
| | | | | JP | H09500363 | A | 14-01-1997 |
| | | | | WO | 9426307 | A1 | 24-11-1994 |
| CN 1869207 | A | | 29-11-2006 | CN | 1869207 | A | 29-11-2006 |
| | | | | WO | 2007134538 | A1 | 29-11-2007 |

EPO FORM P0459

For more details about this annex : see Official Journal of the European Patent Office, No. 12/82

## ANNEX TO THE EUROPEAN SEARCH REPORT
## ON EUROPEAN PATENT APPLICATION NO.

EP 16 16 5526

This annex lists the patent family members relating to the patent documents cited in the above-mentioned European search report.
The members are as contained in the European Patent Office EDP file on
The European Patent Office is in no way liable for these particulars which are merely given for the purpose of information.

11-07-2016

| Patent document cited in search report | | | Publication date | Patent family member(s) | | | Publication date |
|---|---|---|---|---|---|---|---|
| US 2003099653 | A1 | | 29-05-2003 | US | 6030615 | A | 29-02-2000 |
| | | | | US | 6080407 | A | 27-06-2000 |
| | | | | US | 6645493 | B1 | 11-11-2003 |
| | | | | US | 2003099653 | A1 | 29-05-2003 |
| | | | | US | 2004053843 | A1 | 18-03-2004 |
| | | | | US | 2006062788 | A1 | 23-03-2006 |
| | | | | US | 2010184091 | A1 | 22-07-2010 |
| | | | | US | 2012028281 | A1 | 02-02-2012 |
| | | | | US | 2012149044 | A1 | 14-06-2012 |
| WO 2007075270 | A2 | | 05-07-2007 | AU | 2006330051 | A1 | 05-07-2007 |
| | | | | CA | 2633486 | A1 | 05-07-2007 |
| | | | | CN | 101374546 | A | 25-02-2009 |
| | | | | CN | 103242451 | A | 14-08-2013 |
| | | | | CN | 105709237 | A | 29-06-2016 |
| | | | | EP | 1959993 | A2 | 27-08-2008 |
| | | | | HK | 1117761 | A1 | 20-03-2015 |
| | | | | JP | 5090366 | B2 | 05-12-2012 |
| | | | | JP | 2009519931 | A | 21-05-2009 |
| | | | | KR | 20080097995 | A | 06-11-2008 |
| | | | | SG | 153825 | A1 | 29-07-2009 |
| | | | | WO | 2007075270 | A2 | 05-07-2007 |
| WO 9817314 | A1 | | 30-04-1998 | AT | 354372 | T | 15-03-2007 |
| | | | | AU | 740478 | B2 | 08-11-2001 |
| | | | | AU | 5101498 | A | 15-05-1998 |
| | | | | CA | 2267069 | A1 | 30-04-1998 |
| | | | | DE | 69737397 | T2 | 07-02-2008 |
| | | | | DK | 0954334 | T3 | 11-06-2007 |
| | | | | EP | 0954334 | A1 | 10-11-1999 |
| | | | | ES | 2283013 | T3 | 16-10-2007 |
| | | | | JP | 2001502698 | A | 27-02-2001 |
| | | | | JP | 2009155335 | A | 16-07-2009 |
| | | | | PT | 954334 | E | 31-05-2007 |
| | | | | US | 6774227 | B1 | 10-08-2004 |
| | | | | US | 2004156848 | A1 | 12-08-2004 |
| | | | | US | 2008317759 | A1 | 25-12-2008 |
| | | | | WO | 9817314 | A1 | 30-04-1998 |
| WO 2011090492 | A1 | | 28-07-2011 | AU | 2010343304 | A1 | 05-07-2012 |
| | | | | CA | 2787074 | A1 | 28-07-2011 |
| | | | | CN | 102725000 | A | 10-10-2012 |
| | | | | EP | 2525820 | A1 | 28-11-2012 |
| | | | | JP | 5786204 | B2 | 30-09-2015 |
| | | | | JP | 2013516996 | A | 16-05-2013 |
| | | | | WO | 2011090492 | A1 | 28-07-2011 |

EPO FORM P0459

For more details about this annex : see Official Journal of the European Patent Office, No. 12/82

## ANNEX TO THE EUROPEAN SEARCH REPORT
## ON EUROPEAN PATENT APPLICATION NO.

EP 16 16 5526

This annex lists the patent family members relating to the patent documents cited in the above-mentioned European search report.
The members are as contained in the European Patent Office EDP file on
The European Patent Office is in no way liable for these particulars which are merely given for the purpose of information.

11-07-2016

| Patent document cited in search report | Publication date | Patent family member(s) | Publication date |
|---|---|---|---|
| US 2011158905 A1 | 30-06-2011 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 6645493 B
- US 200310235584 A

- WO 2009086920 A

### Non-patent literature cited in the description

- **BLOOM et al.** *Science,* 1966, vol. 153, 80-2
- **DAVID et al.** *PNAS,* 1966, vol. 56, 72-7
- **WEISER et al.** *PNAS,* 1989, vol. 86, 7522-6
- **SUN et al.** *PNAS,* 1996, vol. 93, 5191-5196
- **CALANDRA et al.** *Nature,* 1995, vol. 377, 68-71
- **BAUGH et al.** *Crit Care Med,* 2002, vol. 30, 27-35
- **MITCHELL, R.A.** *Cellular Signalling,* 2004, vol. 16 (1), 13-19
- **LUE, H. et al.** *Oncogene,* 2007, vol. 26 (35), 5046-59
- **NISHIHIRA et al.** *J Interferon Cytokine Res.,* 2000, vol. 20, 751-62
- **SHIMIZU et al.** *FEBS Lett.,* 1996, vol. 381, 199-202
- **KAWAGUCHI et al.** *Leukoc. Biol.,* 1986, vol. 39, 223-232
- **WEISER et al.** *Cell. Immunol.,* 1985, vol. 90, 167-78
- **CALANDRA et al.** *J. Inflamm.,* 1995, vol. 47, 39-51
- **GALAT et al.** *Eur. J. Biochem,* 1994, vol. 224, 417-21
- **WATARAI et al.** *PNAS,* 2000, vol. 97, 13251-6

- General Principles of Cancer Chemotherapy. Introduction. **GOODMAN ; GILMAN.** The Pharmacological Basis of Therapeutics
- **SAMBROOK et al.** Molecular Cloning: A Laboratory Manual. Cold Spring Harbor Laboratory Press, 1989
- **AUSUBEL et al.** Current Protocols in Molecular Biology. Greene Publishing Associates, 1992
- **HARLOW ; LANE.** Antibodies: A Laboratory Manual. Cold Spring Harbor Laboratory Press, 1990
- Fundamental Immunology. Raven Press, 1989
- **KABAT.** Sequences of Proteins of Immunological Interest. National Institutes of Health, 1987
- **CHOTHIA et al.** *J. Mol. Biol.,* 1987, vol. 196, 901-917
- **CHOTHIA et al.** *Nature,* 1989, vol. 342, 878-883
- **ANGAL et al.** *Mol Immunol.,* 1993, vol. 30, 105-108
- General Principles of Cancer Chemotherapy. **GOODMAN ; GILMAN.** The Pharmacological Basis of Therapeutics
- General Principles of Cancer Chemotherapy. Introduction. The Pharmacological Basis of Therapeutics